# EUROPEAN PATENT APPLICATION

(11) **EP 4 321 499 A1**
(43) Date of publication of application: **14.02.2024**
(21) Application number: 22784111.1
(22) Date of filing: 07.04.2022
(51) Int. Cl.: C07B 59/00, C07C 15/14, C07C 319/20, C07D 307/28, C07D 213/127

(54) **METHOD FOR PREPARING DEUTERATED CHEMICAL BY MEANS OF DEUTERATION REACTION OF CARBON-HYDROGEN BOND WITH DEUTERIUM GAS UNDER CATALYSIS OF ALKALI**

(30) Priority: 09.04.2021 CN 202110381786; 15.04.2021 CN 202110406555; 30.04.2021 CN 202110479540; 25.10.2021 CN 202111239237; 25.10.2021 CN 202111239460
(71) Applicant: Fudan University, Shanghai 200438 (CN)
(72) Inventor: GUAN, Bingtao, Shanghai 200438 (CN); DU, Huizhen, Shanghai 200438 (CN)
(74) Representative: Barker Brettell LLP
(86) International application number: PCT/CN2022/085660
(87) International publication number: WO 2022/214045

(57) **Abstract**

The present application provides a method for preparing a deuterated chemical by means of a deuteration reaction of a carbon-hydrogen bond with a deuterium gas under the catalysis of an alkali, wherein in the presence of a catalyst, a deuterium gas is added into a compound containing a carbon-hydrogen bond for a deuteration reaction so as to generate a deuterated compound. A deuterium gas is used as a deuterium source, such that multiple water separation operations, tedious steps and the wasting of energy caused by usage of a large amount of deuterium oxide as a deuterium source are avoided. Moreover, a cheap and easily available alkali metal compound is used for replacing an expensive transition metal catalyst and a complex-structure ligand as a catalyst for a deuteration reaction, and the alkali metal compound has the advantages of a low cost, a good compatibility with functional groups of a substrate and a high deuteration rate. The present application provides a new, low-cost, green and efficient deuteration method, which has a high application value.

## Description

This application claims priority of the Chinese patent applications filed with China National Intellectual Property Administration (CNIPA) on April 9, 2021, with application number 202110381786.6 titled "A Deuteration Method for Benzylic Carbon-Hydrogen Bonds," filed with CNIPA on April 15, 2021, with application number 202110406555.6 titled "A Deuteration Method for Methyl Sulfide Compounds," filed with CNIPA on April 30, 2021, with application number 202110479540.2 titled "A Deuteration Method for sp² Hybridized C-H Bonds," filed with CNIPA on October 25, 2021, with application number 202111239237.1 titled "A Novel Deuteration Method for Pyridine and its Derivatives," and filed with CNIPA on October 25, 2021, with application number 202111239460.6 titled "A Novel Deuteration Method for Sulfones, Sulfoxides, Sulfonamides, and Sulfinamides Compounds," the entirety of which are incorporated by reference herein.

### TECHNICAL FIELD

This application relates to the field of organic synthesis and, more specifically, to a method for preparing deuterated chemicals through a deuteration reaction of a carbon-hydrogen bond with deuterium gas under catalysis of alkali.

### BACKGROUND OF THE INVENTION

Deuterated compounds, as a class of high-value-added compounds, have wide applications in organic synthesis, mechanistic studies, drug metabolism, and drug modification. In recent years, the development of new, mild, efficient, and universally applicable deuteration methods has garnered significant attention from chemists.

With the advancement of C-H bond activation reactions, transition metal-catalyzed hydrogen-deuterium exchange reactions have been widely used for the synthesis of deuterated compounds. This method typically requires expensive transition metal catalysts or complex ligands and is commonly employed for the selective deuteration reaction of C(sp²)-H bonds in aromatic and heteroaromatic compounds containing directing groups. There has been less research on the selective hydrogen-deuterium exchange reactions of C(sp²)-H and C(sp³)-H bonds in aromatic compounds without directing groups.

On the other hand, current deuteration reactions often involve heating to prepare deuterated compounds in the presence of a large quantity of deuterium water. However, due to the equilibrium of the hydrogen-deuterium exchange reaction, it is more economical to remove the resulting water and replace it with fresh deuterium water after multiple cycles of chemical equilibrium to achieve a deuterated product with a high deuteration rate. This method requires a significant amount of deuterium water as a deuterium source and involves numerous water separation operations, leading to cumbersome steps and substantial energy waste. Therefore, there is an urgent need for the development of a simple, efficient, and cost-effective method for carbon-hydrogen bond deuteration.

### SUMMARY OF THE INVENTION

The purpose of this application is to provide a simple, efficient, and cost-effective method for preparing deuterated chemical compounds through a deuteration reaction of a carbon-hydrogen bond with deuterium gas under catalysis of alkali. The specific technical solution is as follows:
This application provides a method for the deuteration of carbon-hydrogen bonds, comprising the following steps:
In the presence of a catalyst, introducing deuterium gas into a compound containing carbon-hydrogen bonds for deuteration to produce deuterated compounds:
wherein, said catalyst is selected from at least one of alkali metal hydroxides, alkali metal alkoxides, alkali metal hydrides, alkali metal hydrocarbon compounds, and alkali metal amino compounds.

In one embodiment of this application, said alkali metal hydroxide is selected from at least one of lithium hydroxide, sodium hydroxide, potassium hydroxide, rubidium hydroxide, and cesium hydroxide;
said alkali metal alkoxide is selected from at least one of lithium methoxide, lithium ethoxide, lithium isopropoxide, lithium tert-butoxide, sodium methoxide, sodium ethoxide, sodium isopropoxide, sodium tert-butoxide, potassium methoxide, potassium ethoxide, potassium isopropoxide, potassium tert-butoxide, alkoxyl rubidium, and alkoxyl cesium; said alkoxyl rubidium is selected from at least one of rubidium methoxide, rubidium ethoxide, rubidium isopropoxide, and rubidium tert-butoxide; said alkoxyl cesium is selected from at least one of cesium methoxide, cesium ethoxide, cesium isopropoxide, and cesium tert-butoxide.

Said alkali metal hydride is selected from at least one of lithium hydride, sodium hydride, potassium hydride, rubidium hydride, and cesium hydride;
said alkali metal hydrocarbon compound is selected from at least one of methyl lithium, trimethylsilylmethyl lithium, ethyl lithium, n-butyl lithium, sec-butyl lithium, tert-butyl lithium, benzyl lithium, phenyl lithium, benzyl sodium, benzyl potassium, benzyl rubidium, and benzyl cesium;
said alkali metal amino compound is selected from at least one of lithium amide, lithium dimethylamide, lithium diethylamide, lithium diisopropylamide, lithium bis(trimethylsilyl)amide, lithium tetramethylpiperidide, sodium amide, sodium diisopropylamide, sodium bis(trimethylsilyl)amide, potassium amide, potassium dimethylamide, potassium diethylamide, potassium diisopropylamide, potassium bis(trimethylsilyl)amide, potassium tetramethylpiperidide, rubidium dimethylamide, rubidium diethylamide, rubidium diisopropylamide, rubidium bis(trimethylsilyl)amide, rubidium tetramethylpiperidide, cesium dimethylamide, cesium diethylamide, cesium diisopropylamide, cesium bis(trimethylsilyl)amide, and cesium tetramethylpiperidide.

In this application, said alkoxyl rubidium or alkoxyl cesium can be prepared by reacting rubidium hydroxide or cesium hydroxide with methanol, ethanol, isopropanol, or tert-butanol.

In one embodiment of this application, wherein:
said alkali metal amino compound is synthesized in situ using an alkali metal hydrocarbon compound and an amine, or using lithium amide and an alkali metal salt; said alkali metal amino compound is selected from at least one of sodium diisopropylamide, potassium dimethylamide, potassium diethylamide, potassium diisopropylamide, potassium bis(trimethylsilyl)amide, potassium tetramethylpiperidide, rubidium dimethylamide, rubidium diethylamide, rubidium diisopropylamide, rubidium bis(trimethylsilyl)amide, rubidium tetramethylpiperidide, cesium dimethylamide, cesium diethylamide, cesium diisopropylamide, cesium bis(trimethylsilyl)amide, and cesium tetramethylpiperidide;
wherein, said alkali metal hydrocarbon compound is selected from at least one of butyl lithium, benzyl potassium, benzyl rubidium, and benzyl cesium;
said amine is selected from at least one of dimethylamine, diethylamine, diisopropylamine, bis(trimethylsilyl)amine, and tetramethylpiperidide;
said lithium amide is selected from at least one of lithium dimethylamide, lithium diethylamide, lithium diisopropylamide, lithium bis(trimethylsilyl)amide, and lithium tetramethylpiperidide;
said alkali metal salt is selected from at least one of sodium salt, potassium salt, rubidium salt, and cesium salt; said sodium salt is selected from sodium hydride and sodium tert-butoxide; said potassium salt is selected from potassium hydride and potassium tert-butoxide; said rubidium salt is selected from at least one of rubidium fluoride, rubidium chloride, rubidium bromide, rubidium iodide, rubidium nitrate, rubidium sulfate, rubidium carbonate, rubidium formate, rubidium acetate, and rubidium perchlorate; said cesium salt is selected from at least one of cesium fluoride, cesium chloride, cesium bromide, cesium iodide, cesium nitrate, cesium sulfate, cesium carbonate, cesium formate, cesium acetate, cesium trifluoroacetate, and cesium perchlorate.

In this application, a mixture of alkali metal hydrocarbon compound and amine, or alkali metal salt and lithium amide, is added to the reaction system of said deuteration reaction, alkali metal amino compounds are synthesized in situ. Under the catalytic action of the in situ-synthesized alkali metal amino compounds, the deuteration reaction is carried out.

It should be noted that alkali metal amino compounds can be added directly to the reaction system of the deuteration reaction as finished catalysts for catalysis, or they can be synthesized in situ in the reaction system of the deuteration reaction for catalysis.

In this application, alkali metal amino compounds are synthesized in situ in said reaction system of the deuteration reaction by acid-base reactions between alkali metal hydrocarbon compounds and amines. Under the catalytic action of the in situ-synthesized alkali metal amino compounds, said deuterium exchange reaction is carried out.

Wherein, said alkali metal benzyl compounds are obtained by the reaction of butyl lithium, toluene and alkali metal tert-butoxide salts; said alkali metal tert-butoxide salts are selected from at least one of potassium tert-butoxide, rubidium tert-butoxide, and cesium tert-butoxide. For example, benzyl potassium is obtained by the reaction of butyl lithium, toluene and potassium tert-butoxide, and benzyl rubidium is obtained by the reaction of butyl lithium, toluene and rubidium tert-butoxide.

In this application, alkali metal amino compounds are synthesized in situ in the reaction system of said deuteration reaction by salt exchange reactions between alkali metal salts and lithium amide. Then, under the catalytic action of the in situ-synthesized alkali metal amino compounds, said deuteration reaction is carried out.

For example, a mixture of sodium tert-butoxide (NaOtBu) or potassium tert-butoxide (KOtBu) and lithium diisopropylamide (LDA) is added to the reaction system of said deuteration reaction, resulting in the in situ synthesis of sodium diisopropylamide (NaDA) or potassium diisopropylamide (KDA). Then, under the catalytic action of the in situ-synthesized NaDA or KDA, said deuteration reaction is carried out.

For example, a mixture of rubidium fluoride (RbF) and lithium bis(trimethylsilyl)amide (LiHMDS) is added to the reaction system of said deuteration reaction, leading to the in situ synthesis of rubidium bis(trimethylsilyl)amide (RbHMDS). Then, under the catalytic action of the in situ-synthesized RbHMDS, said deuteration reaction is carried out.

A mixture of cesium salts, such as cesium chloride (CsCI), cesium carbonate (Cs₂CO₃), cesium trifluoroacetate (CsTFA), or cesium perchlorate (CsClO₄), and lithium bis(trimethylsilyl)amide (LiHMDS) is added to the reaction system of said deuteration reaction, resulting in the in situ synthesis of cesium bis(trimethylsilyl)amide (CsHMDS). Then, under the catalytic action of the in situ-synthesized CsHMDS, said deuteration reaction is carried out.

For another example, a mixture of butyl lithium and tetramethylpiperidine is added to the reaction system of said deuteration reaction, leading to the in situ synthesis of lithium tetramethylpiperidide (TMPLi). Then, under the catalytic action of the in situ-synthesized TMPLi, said deuteration reaction is carried out.

For another example, a mixture of cesium fluoride (CsF) and lithium tetramethylpiperidide (TMPLi) is added to said reaction system of the deuteration reaction, resulting in the in situ synthesis of cesium tetramethylpiperidide (TMPCs). Then, under the catalytic action of the in situ-synthesized TMPCs, said deuteration reaction is carried out.

The above methods for in situ synthesis of catalysts are simple to operate and the above in situ-synthesized alkali metal amino compounds provide excellent catalytic effects for said deuteration reaction.

In this application, the alkali metal amino compound can also be prepared according to the method of in situ synthesis of alkali metal amino compound by the acid-base reaction between a mixture of alkali metal hydrocarbon compound and amine or the salt exchange reaction between a mixture of alkali metal salt and lithium amide. The alkali metal amino compound is then obtained through separation and purification. Subsequently, the prepared alkali metal amino compound is introduced into the reaction system of said deuteration reaction to carry out said deuteration reaction.

In one embodiment of this application, said compounds containing carbon-hydrogen bonds are selected from at least one of the following: aromatic compounds with carbon-hydrogen bonds at the benzyl position; thioether compounds with carbon-hydrogen bonds at the α position; sulfone, sulfoxide, sulfonamide, and sulfinamide compounds with carbon-hydrogen bonds at the α position; aryl ether compounds, fluorobenzene compounds; aromatic hydrocarbons, heteroaromatic hydrocarbons with aromatic carbon-hydrogen bonds, and alkene compounds with alkenyl carbon-hydrogen bonds.

In this application, said aromatic hydrocarbon compounds with carbon-hydrogen bonds at the benzyl position include but are not limited to toluene, alkylbenzenes, alkylpyridines, alkylfurans, alkylthiophenes, alkylpyrroles, benzyl ethers, benzyl thioethers, benzylamines, or benzylsilane compounds.

Said thioether compounds with carbon-hydrogen bonds at the α position include but are not limited to methyl aryl thioethers, methyl alkyl thioethers, or dialkyl thioethers.

Said sulfone, sulfoxide, sulfonamide, and sulfinamide compounds with carbon-hydrogen bonds at the α position include but are not limited to dimethyl sulfoxide, phenyl methyl sulfoxide, dialkyl sulfoxides, alkyl aryl sulfoxides, dialkyl sulfones, alkyl aryl sulfones, *N,N-*dimethyl sulfonamide, alkyl sulfonamides, or alkyl sulfinamides.

Said aryl ether compounds with aromatic carbon-hydrogen bonds include but are not limited to phenyl methyl ether, dimethoxybenzene, 1,3,5-trimethoxybenzene, diphenyl ether, 1,2-methylenedioxybenzene, benzodioxane, or alkoxypyridine.

Said fluorobenzene compounds include but are not limited to fluorobenzene, difluorobenzene, fluoronaphthalene or alkyl fluorobenzene.

Said aromatic hydrocarbons include but are not limited to benzene, biphenyl, naphthalene, anthracene, phenanthrene, pyrene, perylene, triphenylamine, or N-phenylcarbazole.

Said heteroaromatic hydrocarbons compounds include but are not limited to pyridine, phenylpyridine, bipyridine, furan, thiophene, pyrrole, benzofuran, benzothiophene, indole, carbazole, imidazole, oxazole or thiazole.

Said alkenes with alkenyl carbon-hydrogen bonds include but are not limited to methylstyrene, allylbenzene, 2,3-dihydrofuran, or 1,4-dioxene.

In one embodiment of this application, said deuteration reaction is as shown in any one of Reaction Equations 1-5.

Reaction Equation 1 is as follows:

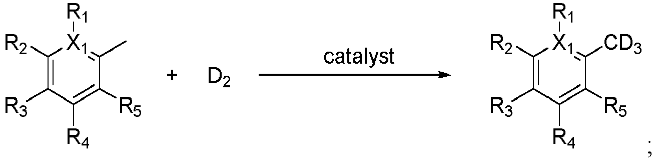

Reaction Equation 2 is as follows:

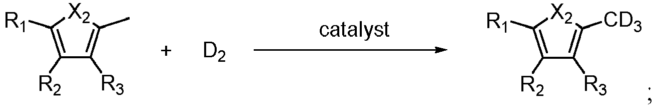

R₁-R₅ are independently selected from any one of H, C₁-C₁₀ hydrocarbon group, C₆-C₂₀ aryl group, halogens, C₁-C₁₀ hydrocarboxyl group, C₃-C₉ silyl group, or C₁-C₂₀ amino group. Two adjacent groups in R₁-R₃ can be connected into a ring. X₁ is a carbon atom or a nitrogen atom, and X₂ is an oxygen atom, a sulfur atom, or a nitrogen atom.

Reaction Equation 3 is as follows:

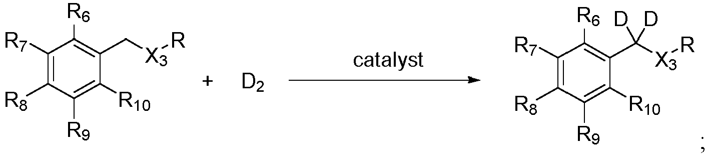

Reaction Equation 4 is as follows:

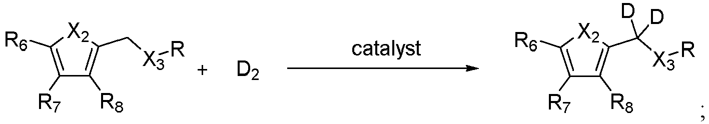

R₆-R₁₀ are independently selected from any one of H, C₁-C₁₀ hydrocarbon group, C₆-C₂₀ aryl group, halogen, C₁-C₁₀ hydrocarboxyl group, or C₁-C₂₀ amino group. Two adjacent groups in R₆-R₈ can be connected into a ring. X₂ is an oxygen atom, a sulfur atom, or a nitrogen atom, and X₃ is any one of a silicon atom, an oxygen atom, a sulfur atom, a carbon atom, or a nitrogen atom. R is a C₁-C₁₀ hydrocarbon group or a C₆-C₂₀ aryl group.

Reaction Equation 5 is as follows:

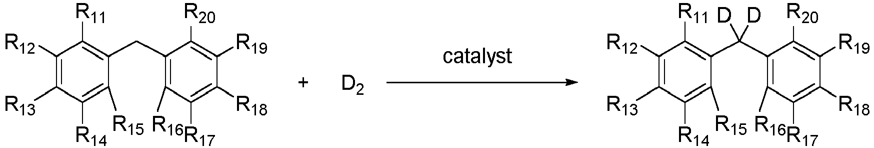

R₁₁-R₂₀ are independently selected from any one of H, C₁-C₁₀ hydrocarbon group, C₆-C₂₀ aryl group, halogen, C₁-C₁₀ hydrocarboxyl group, or C₁-C₂₀ amino group.

It should be noted that when the compound containing carbon-hydrogen bonds is selected from aromatic hydrocarbon compounds with carbon-hydrogen bonds at the benzyl position, i.e., the substrate of the deuteration reaction contains benzyl carbon-hydrogen bonds, said deuteration reaction includes but is not limited to the deuteration reactions shown in Reaction Equations 1 to Reaction Equation 5, and the carbon-hydrogen bonds that are deuterated are selected from the C(sp³)-H bonds at the benzyl position of the aromatic hydrocarbon compounds containing carbon-hydrogen bonds at the benzyl position.

In one embodiment of this application, said deuteration reaction is as shown in any one of Reaction Equations 6-7.

Reaction Equation 6 is as follows:

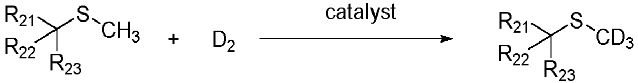

R₂₁-R₂₃ are independently selected from any one of H, C₁-C₁₀ hydrocarbon group, C₆-C₂₀ aryl group, C₃-C₉ silyl group, C₁-C₁₀ alkyloxyl group, or C₁-C₂₀ amino group. R₂₁-R₂₃ can be connected into an aliphatic hydrocarbon ring or an aromatic ring.

Reaction Equation 7 is as follows:

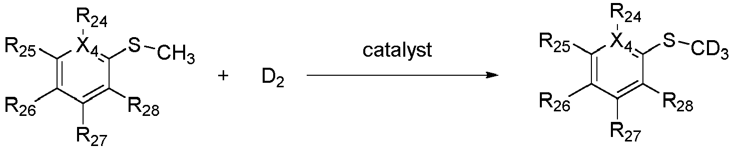

R₂₄-R₂₈ are independently selected from any one of H, C₁-C₁₀ hydrocarbon group, C₆-C₂₀ aryl group, halogen, C₁-C₁₀ hydrocarboxyl group, C₁-C₁₀ hydrocarbon sulfur group, or C₁-C₂₀ amino group. X₄ is a carbon atom or a nitrogen atom.

It should be noted that when the compound containing carbon-hydrogen bonds is selected from thioether compounds with carbon-hydrogen bonds at the α position, said deuteration reaction includes but is not limited to the deuteration reactions shown in Reaction Equation 6-7, and the carbon-hydrogen bonds that are deuterated are selected from at least one of the C(sp³)-H bonds at the α position directly connected to sulfur atoms in the thioether compounds with carbon-hydrogen bonds at the α position.

In one embodiment of this application, the catalyst in Reaction Equation 1-7 is selected from at least one of potassium diisopropylamide, potassium bis(trimethylsilyl)amide, potassium tetramethylpiperidide, rubidium diisopropylamide, rubidium bis(trimethylsilyl)amide, rubidium tetramethylpiperidide, cesium diisopropylamide, cesium bis(trimethylsilyl)amide, and cesium tetramethylpiperidide.

In one embodiment of this application, said deuteration reaction is as shown in any one of Reaction Equations 8-12.

Reaction Equation 8 is as follows:

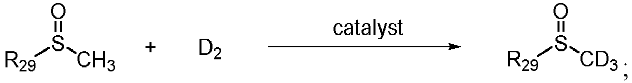

Reaction Equation 9 is as follows:

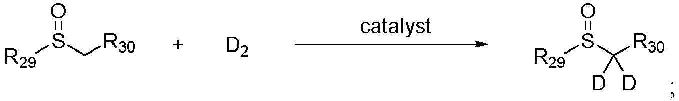

Reaction Equation 10 is as follows:

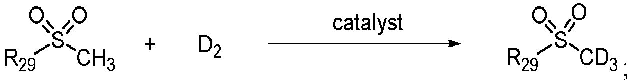

Reaction Equation 11 is as follows:

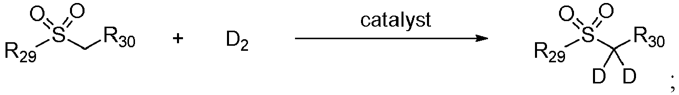

Reaction Equation 12 is as follows:

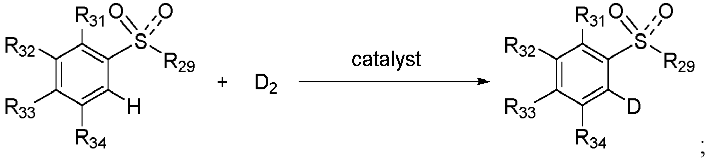

R₂₉ is independently selected from any one of C₁-C₁₀ hydrocarbon group, C₆-C₂₀ aryl group, C₁-C₁₀ alkyloxyl group, or C₁-C₂₀ amino group. R₃₀ is independently selected from any one of C₁-C₁₀ hydrocarbon group or C₆-C₂₀ aryl group, and R₂₉ and R₃₀ can be connected into a ring. R₃₁-R₃₄ are independently selected from any one of H, C₁-C₁₀ hydrocarbon group, C₆-C₂₀ aryl group, C₁-C₁₀ alkyloxyl group, or C₁-C₂₀ amino group, and two adjacent groups in R₃₁-R₃₄ can be connected into a ring.

It should be noted that when said compound containing carbon-hydrogen bonds is selected from said compounds with carbon-hydrogen bonds at the α position in sulfones, sulfoxides, sulfonamides, and sulfinamides, and in aromatic sulfones, sulfoxides, sulfonamides, and sulfinamides, i.e., when the reaction substrates in the deuteration reaction contains an S=O structure, said deuteration reaction includes but is not limited to the deuteration reactions as shown in any one of Reaction Equations 8-12, and the carbon-hydrogen bonds that are deuterated are selected from at least one of the C(sp³)-H bonds directly connected to sulfur atoms in the compounds containing carbon-hydrogen bonds and the C(sp²)-H bonds on the benzene ring with a group containing an S=O structure as the directing group. Wherein, the above group containing the S=O structure is selected from at least one of the sulfones, sulfoxides, sulfonamide, or sulfinamide groups in the reaction substrates mentioned above.

In one embodiment of this application, the catalyst in Reaction Equations 8-11 is selected from at least one of potassium tert-butoxide, sodium hydroxide, and potassium hydroxide.

In one embodiment of this application, said deuteration reaction is as shown in any one of Reaction Equations 13-15.

Reaction Equation 13 is as follows:

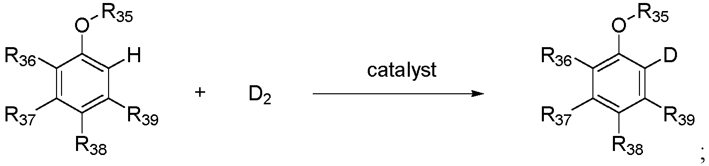

Reaction Equation 14 is as follows:

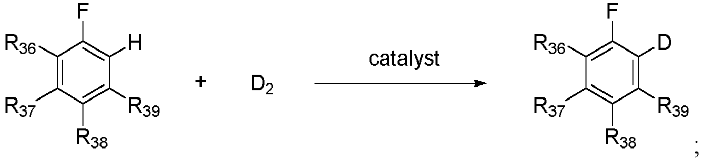

Reaction Equation 15 is as follows:

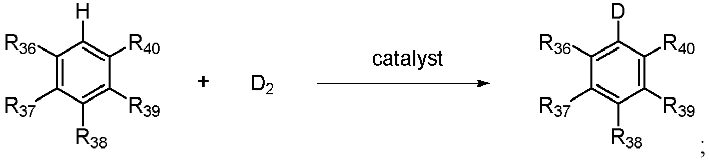

R₃₅-R₄₀ are independently selected from any one of H, C₁-C₁₀ hydrocarbon group, or C₆-C₂₀ aryl group, and two adjacent groups in R₃₅-R₄₀ can be connected into a ring.

In one embodiment of this application, said deuteration reaction is as shown in any one of Reaction Equations 16-17.

Reaction Equation 16 is as follows:

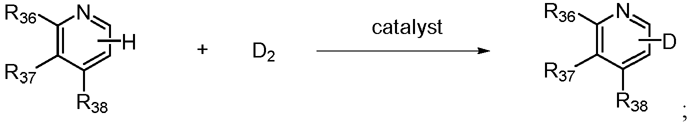

Reaction Equation 17 is as follows:

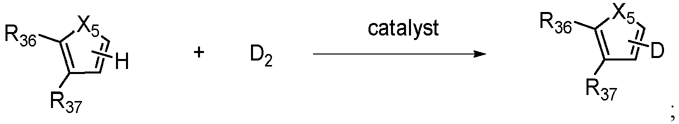

R₃₆-R₃₈ are independently selected from any one of H, C₁-C₁₀ hydrocarbon group, or C₆-C₂₀ aryl group, and two adjacent groups in R₃₆-R₃₈ can be connected into a ring. X₅ is selected from any one of silicon, oxygen, sulfur, carbon, or nitrogen atoms.

It should be noted that said deuteration reaction can occur at any position on aromatic heterocycles such as pyridine, furan, and thiophene.

In one embodiment of this application, said deuteration reaction is as shown in Reaction Equation 18.

Reaction Equation 18 is as follows:

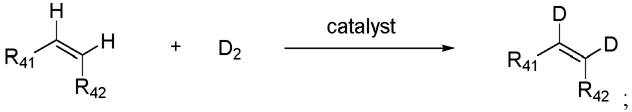

R₄₁ and R₄₂ are independently selected from any one of H, C₁-C₁₀, hydrocarbon group, C₆-C₂₀ aryl group, C₃-C₉ silyl group, C₁-C₁₀ alkoxyl group, or C₁-C₂₀ amino group, and R₄₁ and R₄₂ can be connected into an aliphatic hydrocarbon ring or an aromatic ring.

It should be noted that when the reaction substrate for said deuteration reaction contains a C(sp²)-H bond, said deuteration reaction includes but is not limited to the deuteration reactions as shown in Reaction Equations 12-18, and the carbon-hydrogen bonds that are deuterated are selected from at least one of the C(sp²)-H bonds contained in the above reaction substrates.

In one embodiment of this application, the catalyst in Reaction Equations 13-18 is selected from at least one of potassium diisopropylamide, potassium bis(trimethylsilyl)amide, potassium tetramethylpiperidide, rubidium diisopropylamide, rubidium bis(trimethylsilyl)amide, rubidium tetramethylpiperidide, cesium diisopropylamide, cesium bis(trimethylsilyl)amide, and cesium tetramethylpiperidide.

In this application, the chemical formula of said C₃-C₉ silane groups is -Si Y₁Y₂Y₃, where Y₁, Y₂, and Y₃ are independently selected from C₁-C₃ alkyl groups. The chemical formula of C₁-C₁₀ alkyloxyl groups or C₁-C₁₀ hydrocarboxyl groups is -OY₄, where Y₄ is selected from C₁-C₁₀ alkyl groups or C₆-C₁₀ aryl groups. The chemical formula of C₁-C₂₀ amino groups is -NY₅Y₆, where Y₅ and Y₆ are independently selected from C₁-C₁₀ alkyl groups or C₆-C₁₀ aryl groups.

In one embodiment of this application, the molar amount of said catalyst is 1%-100% of the molar amount of said compound containing carbon-hydrogen bonds.

In one embodiment of this application, the pressure of deuterium gas in said deuteration reaction is 1 bar to 50 bar.

In one embodiment of this application, said deuteration reaction is carried out in an organic solvent or under solvent-free conditions, and said organic solvents are selected from at least one of aromatic hydrocarbon solvents, alkane solvents, and ether solvents. Among them, the aromatic hydrocarbon solvents are selected from at least one of deuterated benzene, benzene, and tert-butylbenzene; the alkane solvents are selected from at least one of n-hexane, cyclohexane, and decalin; the ether solvents are selected from at least one of ether, tetrahydrofuran, methyl tert-butyl ether, methyl cyclopentyl ether, and n-butyl ether.

In this application, the reaction temperature for said deuteration reaction is 40°C-150°C, and the reaction time is 12 hours-96 hours.

In this application, after a period of the deuteration reaction, the gas in the reaction vessel can be replaced with fresh deuterium gas for further reaction, and through two or more such processes, a high deuteration rate of the reactants can be achieved.

In this application, the deuteration reaction is carried out in a sealed container, and an inert atmosphere such as nitrogen or argon can be introduced into the sealed container.

The advantageous effects of this application are as follows:
This application provides a deuteration method for carbon-hydrogen bonds, which, in the presence of a catalyst, involves the addition of deuterium gas to a compound containing a carbon-hydrogen bond to perform a deuteration reaction to generate deuterated compounds. By using deuterium gas as the deuterium source, avoiding the use of a large amount of deuterium oxide as a deuterium source, thus avoiding multiple water removal steps, cumbersome steps, and a large amount of energy waste. Additionally, using inexpensive and readily available alkali metal compounds as catalysts for deuteration reactions, instead of expensive transition metal catalysts and complex ligands, offers advantages of low cost, excellent substrate functional group compatibility, and high deuteration rate. Furthermore, the deuteration method disclosed in this application is characterized by mild reaction conditions, simple operation, and the ability to prepare deuterated products in one step. This deuteration method opens up a new, low-cost, and environmentally friendly pathway for the preparation of deuterated chemicals and pharmaceuticals, which has high practical value.

In addition, in the deuteration method of the present application, the compounds containing C-H bonds, that is, the reaction substrate of the deuteration reaction is selected from at least one of the aromatic compounds with C-H bonds at the benzyl position, thioether compounds with C-H bonds at the alpha position, sulfone, sulfoxide, sulfamide, sulfinamide compounds with C-H bonds at the α position, aryl ether compounds, fluorobenzene compounds, aromatic hydrocarbons, heteroaromatic hydrocarbons with aryl C-H bonds and alkenes with alkenyl C-H bonds. The deuterated C-H bond in the present application relates to at least one of the C(sp³)-H bond at the benzyl position in the above reaction substrate, the C(sp³)-H bond at the α position connected to the sulfur atom on the thioether group, the C(sp³)-H bond at the α position directly connected to the sulfur atom of the sulfone, sulfoxide, sulfamide or sulfinamide group, and the C(sp²)-H bonds in the benzene ring with the sulfone, sulfoxide, sulfamide or sulfinamide group as the directing group and the C(sp²)-H bonds of aryl ethers, fluorobenzenes, aromatic hydrocarbons, heteroaromatic hydrocarbons and alkenes having alkenyl hydrocarbon bonds. In addition, the deuteration method of the present application has good deuteration effect on the above-mentioned reaction substrates and the above-mentioned carbon-hydrogen bonds, and has the advantages of wide application range and high deuteration rate.

Of course, the implementation of any method of this application does not necessarily need to achieve all the advantages described above.

### DESCRIPTION OF DRAWINGS

In order to provide a clearer understanding of the technical solutions in the embodiments of this application, a brief introduction to the drawings required for the embodiments is presented below. It is evident that the following descriptions of the drawings are only some of the embodiments of this application, and those skilled in the art can obtain additional drawings based on these.
Figure 1 is the ¹H NMR spectrum of deuterated 4-phenyltoluene in Example 6;
Figure 2 is the ¹³C NMR spectrum of deuterated 4-phenyltoluene in Example 6;
Figure 3 is the ¹H NMR spectrum of deuterated p-xylene in Example 21;
Figure 4 is the ¹³C NMR spectrum of deuterated p-xylene in Example 21;
Figure 5 is the ¹H NMR spectrum of deuterated 4-ethylbiphenyl in Example 22;
Figure 6 is the ¹³C NMR spectrum of deuterated 4-ethylbiphenyl in Example 22;
Figure 7 is the ¹H NMR spectrum of deuterated 2-methylpyridine in Example 23;
Figure 8 is the ¹³C NMR spectrum of deuterated 2-methylpyridine in Example 23;
Figure 9 is the ¹H NMR spectrum of deuterated 3-methylpyridine in Example 24;
Figure 10 is the ¹³C NMR spectrum of deuterated 3-methylpyridine in Example 24;
Figure 11 is the ¹H NMR spectrum of deuterated 1,2-dimethylindole in Example 25;
Figure 12 is the ¹³C NMR spectrum of deuterated 1,2-dimethylindole in Example 25;
Figure 13 is the ¹H NMR spectrum of deuterated 2-methylbenzothiophene in Example 26;
Figure 14 is the ¹³C NMR spectrum of deuterated 2-methylbenzothiophene in Example 26;
Figure 15 is the ¹H NMR spectrum of deuterated diphenylmethane in Example 27;
Figure 16 is the ¹³C NMR spectrum of deuterated diphenylmethane in Example 27;
Figure 17 is the ¹H NMR spectrum of deuterated benzylmethyl ether in Example 28;
Figure 18 is the ¹³C NMR spectrum of deuterated benzylmethyl ether in Example 28;
Figure 19 is the ¹H NMR spectrum of deuterated benzyl phenyl sulfide in Example 29;
Figure 20 is the ¹³C NMR spectrum of deuterated benzyl phenyl sulfide in Example 29;
Figure 21 is the ¹H NMR spectrum of deuterated N-ethyl-N-benzyl aniline in Example 30;
Figure 22 is the ¹³C NMR spectrum of deuterated N-ethyl-N-benzyl aniline in Example 30;
Figure 23 is the ¹H NMR spectrum of deuterated phenyl methyl sulfide in Example 31;
Figure 24 is the ¹³C NMR spectrum of deuterated phenyl methyl sulfide in Example 31;
Figure 25 is the ¹H NMR spectrum of deuterated methylthiocyclohexyl ester in Example 32;
Figure 26 is the ¹³C NMR spectrum of deuterated methylthiocyclohexyl ester in Example 32;
Figure 27 is the ¹H NMR spectrum of deuterated dimethyl sulfoxide in Example 33;
Figure 28 is the ¹³C NMR spectrum of deuterated dimethyl sulfoxide in Example 33;
Figure 29 is the ¹H NMR spectrum of deuterated phenyl methyl sulfoxide in Example 34;
Figure 30 is the ¹³C NMR spectrum of deuterated phenyl methyl sulfoxide in Example 34;
Figure 31 is the ¹H NMR spectrum of deuterated *N,N-*dimethylmethanesulfonamide in Example 35;
Figure 32 is the ¹³C NMR spectrum of deuterated *N,N-*dimethylmethanesulfonamide in Example 35;
Figure 33 is the ¹H NMR spectrum of deuterated anisole in Example 36;
Figure 34 is the ¹³C NMR spectrum of deuterated anisole in Example 36;
Figure 35 is the ¹H NMR spectrum of deuterated 1,3-benzodioxole in Example 37;
Figure 36 is the ¹³C NMR spectrum of deuterated 1,3-benzodioxole in Example 37;
Figure 37 is the ¹H NMR spectrum of deuterated 4-fluorobiphenyl in Example 38;
Figure 38 is the ¹³C NMR spectrum of deuterated 4-fluorobiphenyl in Example 38;
Figure 39 is the ¹H NMR spectrum of deuterated 1,4-difluorobenzene in Example 39;
Figure 40 is the ¹³C NMR spectrum of deuterated 1,4-difluorobenzene in Example 39;
Figure 41 is the ¹H NMR spectrum of deuterated benzene in Example 40;
Figure 42 is the ¹³C NMR spectrum of deuterated benzene in Example 40;
Figure 43 is the ¹H NMR spectrum of deuterated pyridine in Example 45;
Figure 44 is the ¹³C NMR spectrum of deuterated pyridine in Example 45;
Figure 45 is the ¹H NMR spectrum of deuterated 2-phenylpyridine in Example 46;
Figure 46 is the ¹³C NMR spectrum of deuterated 2-phenylpyridine in Example 46;
Figure 47 is the ¹H NMR spectrum of deuterated 2,2'-bipyridine in Example 47;
Figure 48 is the ¹H NMR spectrum of deuterated N-methylindole in Example 48;
Figure 49 is the ¹³C NMR spectrum of deuterated N-methylindole in Example 48;
Figure 50 is the ¹H NMR spectrum of deuterated benzothiophene in Example 49;
Figure 51 is the ¹³C NMR spectrum of deuterated benzothiophene in Example 49;
Figure 52 is the ¹H NMR spectrum of deuterated β-methylstyrene in Example 50;
Figure 53 is the ¹H NMR spectrum of deuterated 2,3-dihydrofuran in Example 51;
Figure 54 is the ¹H NMR spectrum of deuterated 1,4-dioxene in Example 52;
Figure 55 is the ¹H NMR spectrum of deuterated 1,3-bis-(2,4,6-trimethylphenyl)imidazol-2-ylidene in Example 53.

### PREFERRED EMBODIMENTS OF THE INVENTION

In order to make the objectives, technical solutions, and advantages of the present application clearer and more understandable, specific embodiments are described below with reference to the attached drawings for further illustrating the present application. Obviously, the described embodiments are only part of the embodiments of the present application, not all of them. All other embodiments obtained by those skilled in the art based on the embodiments in the present application are within the scope of protection of the present application.

### EXAMPLES

Below, examples are provided to further illustrate the embodiments of the present application. The chemical structures of deuterated products in the deuteration reactions of the embodiments show the positions of deuterated carbon-hydrogen bonds and the corresponding deuteration rate.

The following abbreviations are used in the examples:

**Table 1**

| Full name | Abbreviation | Full name | Abbreviation |
|---|---|---|---|
| Lithium bis(trimethylsilyl)amide | LiHMDS | Potassium tert-butoxide | KO^{t}Bu |
| Sodium bis(trimethylsilyl)amide | NaHMDS | Potassium hydride | KH |
| Potassium bis(trimethylsilyl)amide | KHMDS | Cesium fluoride | CsF |
| Cesium bis(trimethylsilyl)amide | CsHMDS | Rubidium fluoride | RbF |
| Benzyl potassium | BnK | Cesium carbonate | Cs₂CO₃ |
| Methyl tert-Butyl ether | TBME | Deuterium | D₂ |
| | | Benzene-d₆ | C₆D₆ |

The preparation of the cesium bis(trimethylsilyl)amide (CsHMDS) catalyst used in the following examples includes:
Under an inert atmosphere, 10 mmol of lithium bis(trimethylsilyl)amide (LiHMDS), 10 mmol of cesium fluoride (CsF), and 30 mL of dry n-hexane were added to a reaction flask equipped with a magnetic stirrer. The reaction solution was heated and refluxed for 15 hours. After the reaction was completed, the solvent was evaporated. Then, 30 mL of toluene was added to dissolve cesium bis(trimethylsilyl)amide (CsHMDS), filtered, and the solvent was drained to obtain 2.20 g of white solid, with a yield of 75%.

The preparation of the benzyl potassium (BnK) catalyst used in the following examples includes:
At -78°C, 21 mL of n-butyl lithium ("BuLi, molar concentration 2.4 M) in hexane was slowly added to a suspension of 100 mL of potassium tert-butoxide (KO^{t}Bu, molar quantity 50 mmol) in toluene in a Schlenk flask. The reaction solution was slowly increased to room temperature and stirred at room temperature for 48 hours. After the reaction, the red suspension was filtered to obtain 4.43 g of red solid, which was benzyl potassium (BnK), with a yield of 68%.

The gas chromatography-mass spectrometry (GC-MS) analysis used in the following examples involved dissolving 1-2 mg of the reaction product obtained after the reaction in 1 mL of ethyl acetate, testing using the GCMS-Q2020 NX instrument to determine the molecular weight and abundance of the product, and then calculate deuteration rate using the IsoPat² calculation method (the IsoPat2 calculation method of Reference J. Org. Chem. 2007, 72, 5778-5783. which is incorporated into this application by reference).

### Example 1

### Preparation of deuterated 4-phenyltoluene

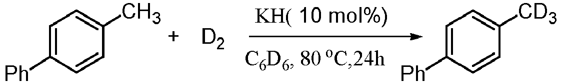

Under an inert atmosphere, in a 25 mL autoclave, 4-phenyltoluene (CAS: 644-08-6) (0.0504 g, 0.3 mmol), deuterated benzene (0.5 mL), and potassium hydride (0.0012 g, 0.03 mmol, 10 mol%) were sequentially added. The autoclave was sealed, filled with deuterium gas to achieve the pressure of deuterium gas in the autoclave is 4 bar, heated at 80°C for 24 hours to undergo the deuteration reaction. After the reaction, the reaction solution was separated by column chromatography, and the obtained deuterated 4-phenyltoluene was subjected to nuclear magnetic resonance ¹HNMR and ¹³C NMR testing, with ¹H NMR performed at a frequency of 400 MHz using deuterated chloroform as the deuterated solvent and ¹³C NMR performed at a frequency of 101 MHz using deuterated chloroform as the deuterated solvent.

The deuteration rate was calculated based on ¹H NMR data, and the deuteration rate of the carbon-hydrogen bond at the benzyl position of deuterated 4-phenyltoluene was determined to be 31%.

### Examples 2 to 20

Except for the preparation parameters and deuteration rates as shown in Table 2, the rest were the same as Example 1.

**Table 2**

| Example | Catalyst and its molar quantity (mol%) | Reaction temperature (°C) | Reaction time (h) | Deuterium gas pressure (bar) | Reaction solvent | Deuteration rate (%) | Column chromatography separation yield (%) |
|---|---|---|---|---|---|---|---|
| 1 | KH (10) | 80 | 24 | 4 | C₆D₆ | 31 | 97 |
| 2 | BnK (10) | 80 | 24 | 4 | C₆D₆ | <10 | 98 |
| 3 | KHMDS (10) | 80 | 24 | 4 | C₆D₆ | 30 | 97 |
| 4 | NaHMDS (10) | 80 | 24 | 4 | C₆D₆ | <5 | 97 |
| 5 | LiHMDS (10) | 80 | 24 | 4 | C₆D₆ | <5 | 97 |
| 6 | CsF (10) + LiHMDS (10) | 80 | 24 | 4 | C₆D₆ | 97 | 97 |
| 7 | RbF (10) + LiHMDS (10) | 80 | 24 | 4 | C₆D₆ | 90 | 98 |
| 8 | Cs₂CO₃ (5) + LiHMDS (10) | 80 | 24 | 4 | C₆D₆ | 75 | 98 |
| 9 | CsF (10) | 80 | 24 | 4 | C₆D₆ | <5 | 97 |
| 10 | CsF (5) + LiHMDS (5) | 80 | 24 | 4 | C₆D₆ | 87 | 97 |
| 11 | CsF (10) + LiHMDS (10) | 80 | 36 | 4 | C₆D₆ | 99 | 99 |
| 12 | CsF (10) + LiHMDS | 100 | 24 | 4 | C₆D₆ | 98 | 99 |
| | (10) | | | | | | |
| 13 | CsHMDS (10) | 80 | 24 | 4 | C₆D₆ | 97 | 98 |
| 14 | CsF (10) + LiHMDS (10) | 80 | 24 | 4 | Benzene | 96 | 96 |
| 15 | CsF (10) + LiHMDS (10) | 80 | 24 | 4 | Tetrahydrofuran | 42 | 96 |
| 16 | CsF (10) + LiHMDS (10) | 80 | 24 | 4 | Ether | 97 | 95 |
| 17 | CsF (10) + LiHMDS (10) | 80 | 24 | 4 | TBME | 97 | 96 |
| 18 | CsF (10) + LiHMDS (10) | 80 | 24 | 4 | n-Hexane | 95 | 95 |
| 19 | CsF (10) + LiHMDS (10) | 80 | 24 | 4 | Cyclohexane | 96 | 96 |
| 20 | CsF (30) + LiHMDS (30) | 80 | 24 | 1 | Benzene | 97 | 97 |

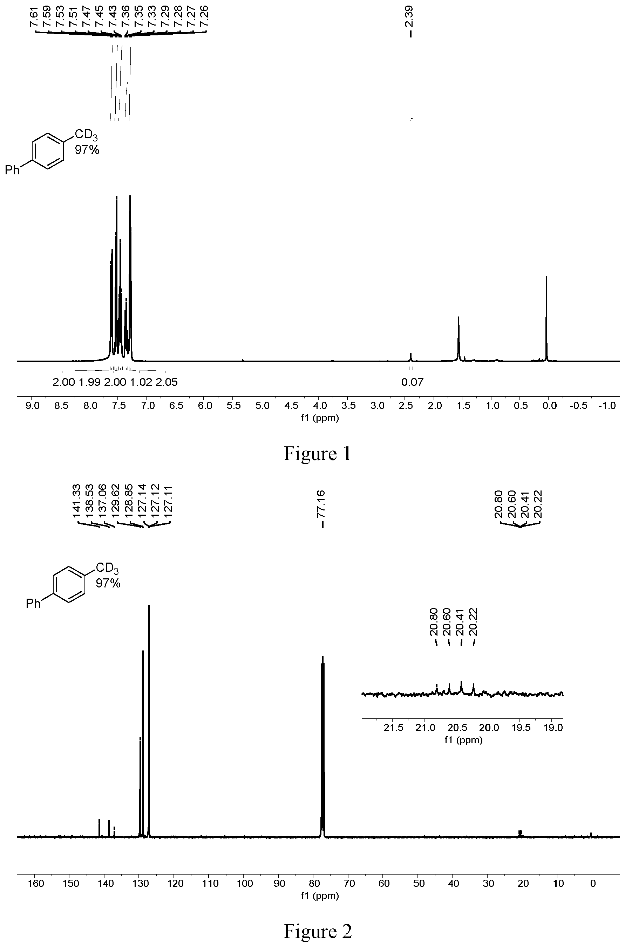

In Example 6, the ¹H NMR spectrum is shown in Figure 1, 1H NMR (400 MHz, CDCl₃) δ 7.64-7.57 (m, 2H), 7.55-7.49 (m, 2H), 7.48-7.41 (m, 2H), 7.38-7.32 (m, 1H), 7.30-7.26 (m, 2H), 2.44-2.33 (m, 0.07H, deuterated positions).The ¹³C NMR spectrum is shown in Figure 2, ¹³C NMR (101 MHz, CDCl₃) δ 141.3, 138.5, 137.1, 129.6, 128.9, 127.14, 127.12, 127.11, 20.8-20.2 (m, 1C, deuterated positions).

### Example 21

### Preparation of Deuterated p-Xylene

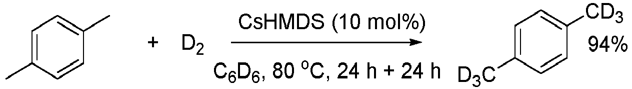

Under an inert atmosphere, in a 25 mL autoclave, p-xylene (CAS: 106-42-3) (0.0319g, 0.3 mmol), deuterated benzene (0.5 mL), and cesium bis(trimethylsilyl)amide (0.0087g, 0.03 mmol, 10 mol%) were sequentially added. The autoclave was sealed, filled with deuterium gas to achieve the pressure of deuterium gas in the autoclave is 4 bar, heated at 80°C for 24 hours to undergo the deuteration reaction. Deuterium gas was then refilled to maintain a pressure of 4 bar for the deuterium gas inside the autoclave, and the reaction was continued for another 24 hours. After completion of the reaction, the reaction solution was directly subjected to ¹H NMR and ¹³C NMR testing.

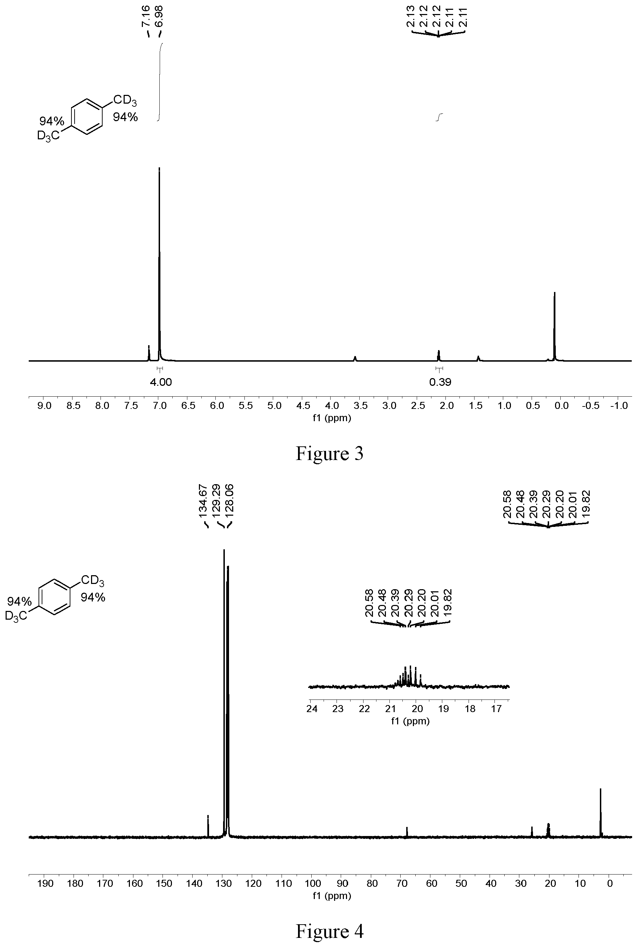

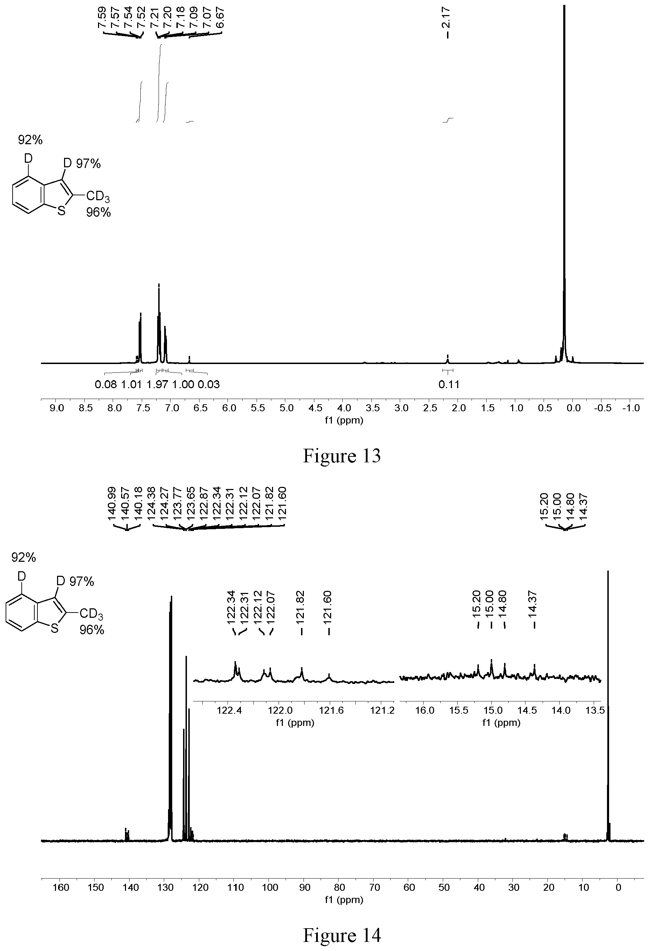

In Example 21, the ¹H NMR spectrum is shown in Figure 3, 1H NMR (400 MHz, C₆D₆) δ 6.98 (s, 4H), 2.17-2.05 (m, 0.39H, deuterated positions). The ¹³C NMR spectrum is shown in Figure 4, 13C NMR (101 MHz, C₆D₆) δ 134.7, 129.3, 20.6-19.8 (m, 2C, deuterated positions).

Based on the ¹H NMR data, the deuteration rate was calculated, and the deuteration rate of the carbon-hydrogen bond at the benzyl position on deuterated p-xylene was determined to be 94%.

### Example 22

### Preparation of Deuterated 4-Ethylbiphenyl

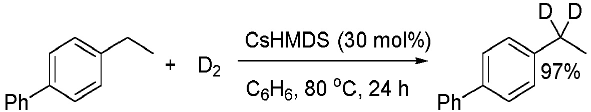

Under an inert atmosphere, in a 25 mL autoclave, 4-ethylbiphenyl (CAS: 5707-44-8) (0.0547 g, 0.3 mmol), benzene (0.5 mL), and cesium bis(trimethylsilyl)amide (0.0264 g, 0.09 mmol, 30 mol%) were added sequentially. The autoclave was sealed, filled with deuterium gas to achieve the pressure of deuterium gas in the autoclave is 4 bar, heated at 80°C for 24 hours to undergo the deuteration reaction. The reaction solution was then separated by column chromatography, with a separation yield of 98%. The obtained product was subjected to ¹H NMR and ¹³C NMR testing using deuterated chloroform (CDCl₃) as the deuterated solvent.

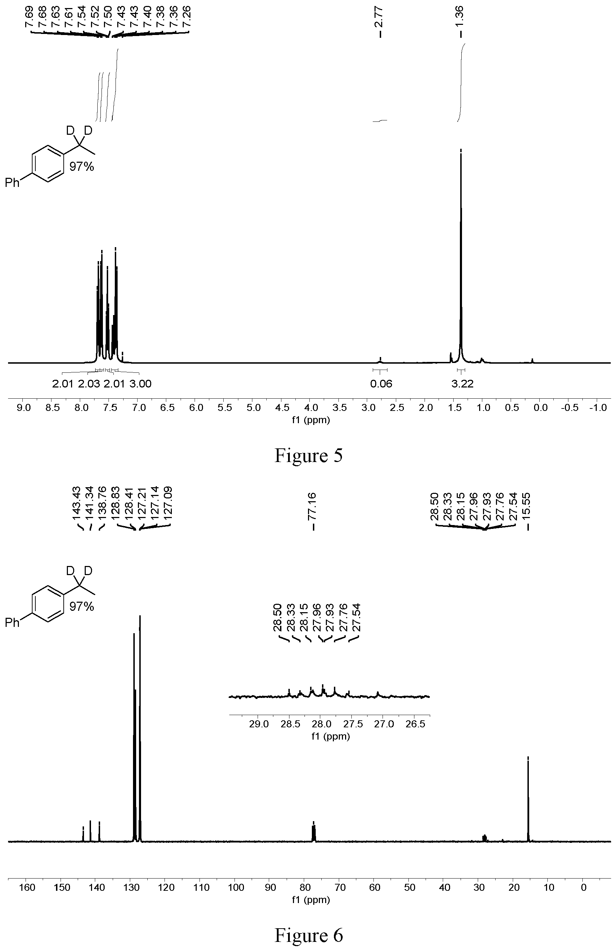

In Example 22, the ¹H NMR spectrum is shown in Figure 5, 1H NMR (400 MHz, CDCl₃) δ 7.73-7.65 (m, 2H), 7.65-7.58 (m, 2H), 7.56-7.48 (m, 2H), 7.45-7.34 (m, 3H), 2.90-2.65 (m, 0.06H, deuterated positions), 1.43-1.30 (m, 3H). The ¹³C NMR spectrum is shown in Figure 6, 13C NMR (101 MHz, CDCl₃) δ 143.4, 141.3, 138.8, 128.8, 128.4, 127.2, 127.14, 127.09, 28.5-27.5 (m, 1C, deuterated positions), 15.6.

Based on the ¹H NMR data, the deuteration rate was calculated, and the deuteration rate of the carbon-hydrogen bond at the benzyl position on deuterated 4-ethylbiphenyl was determined to be 97%.

### Example 23

### Preparation of Deuterated 2-Methylpyridine

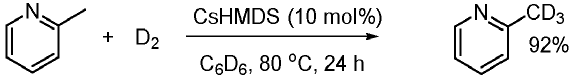

Under an inert atmosphere, in a 25 mL autoclave, 2-methylpyridine (CAS: 109-06-8) (0.0280 g, 0.3 mmol), deuterated benzene (0.5 mL), and cesium bis(trimethylsilyl)amide (0.0087 g, 0.03 mmol, 10 mol%) were added sequentially. The autoclave was sealed, filled with deuterium gas to achieve the pressure of deuterium gas in the autoclave is 4 bar, heated at 80°C for 24 hours to undergo the deuteration reaction. Then, the reaction solution was directly subjected to ¹H NMR and ¹³C NMR testing.

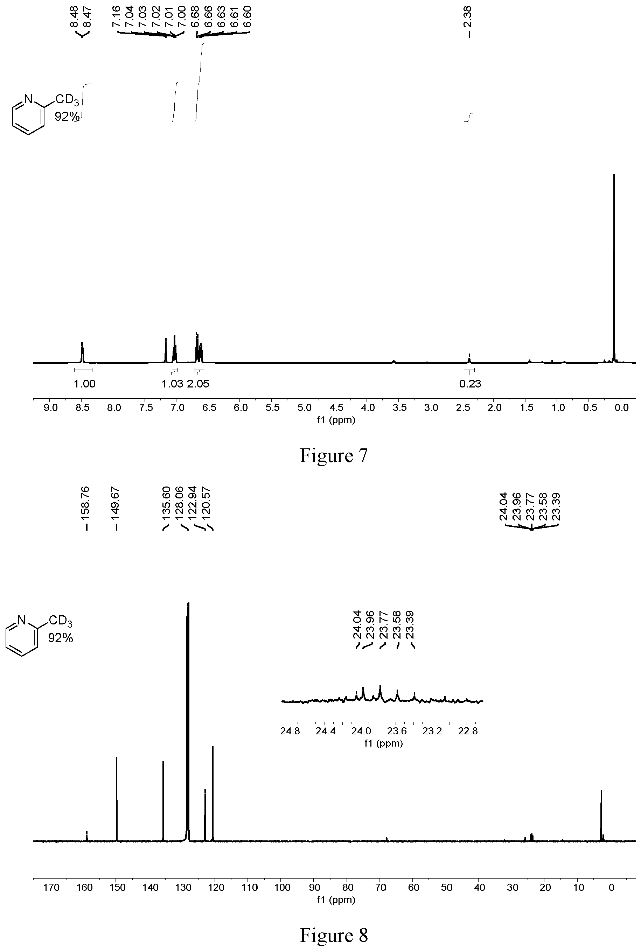

In Example 23, the ¹H NMR spectrum is shown in Figure 7, 1H NMR (400 MHz, C₆D₆) δ 8.61-8.32 (m, 1H), 7.07-6.98 (m, 1H), 6.71-6.57 (m, 2H), 2.47-2.30 (m, 0.23H, deuterated positions). The ¹³C NMR spectrum is shown in Figure 8, 13C NMR (101 MHz, C₆D₆) δ 158.8, 149.7, 135.6, 122.9, 120.6, 24.0-23.4 (m, 1C, deuterated positions).

Based on the ¹H NMR data, the deuteration rate was calculated, and the deuteration rate of the carbon-hydrogen bond at the benzyl position on deuterated 2-methylpyridine was determined to be 92%.

### Example 24

### Preparation of Deuterated 3-Methylpyridine

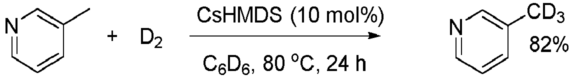

Under an inert atmosphere, in a 25 mL autoclave, 3-methylpyridine (CAS: 108-99-6) (0.0280 g, 0.3 mmol), deuterated benzene (0.5 mL), and cesium bis(trimethylsilyl)amide (0.0087 g, 0.03 mmol, 10 mol%) were added sequentially. The autoclave was sealed, filled with deuterium gas to achieve the pressure of deuterium gas in the autoclave is 4 bar, heated at 80°C for 24 hours to undergo the deuteration reaction. Then, the reaction solution was directly subjected to ¹H NMR and ¹³C NMR testing.

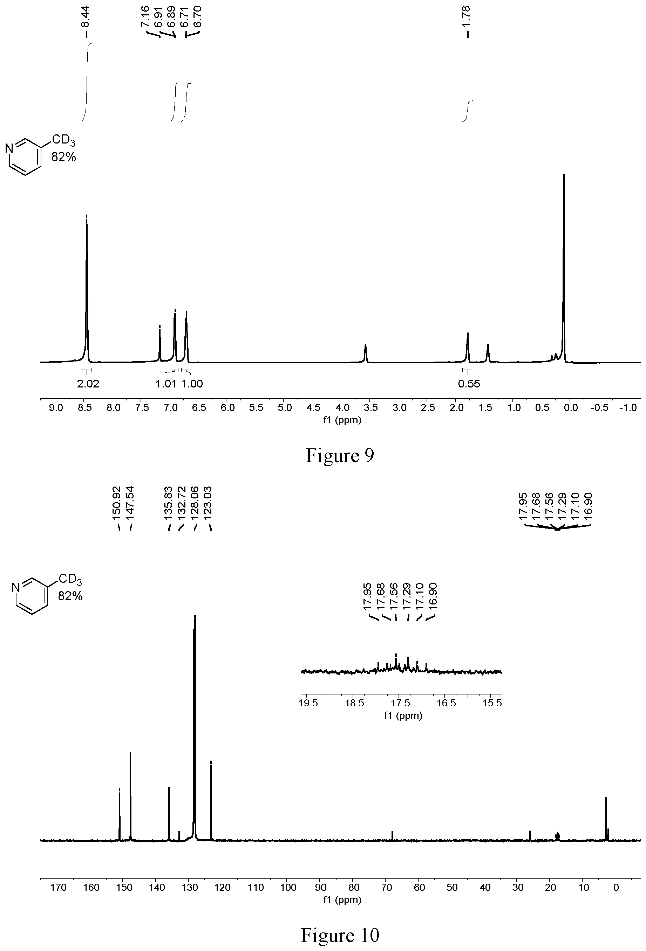

In Example 24, the ¹H NMR spectrum is shown in Figure 9, 1H NMR (400 MHz, C₆D₆) δ 8.44 (s, 2H), 6.90 (d, *J* = 6.4Hz, 1H), 6.79-6.60 (m, 1H), 1.87-1.69 (m, 0.55H, deuterated positions). The ¹³C NMR spectrum is shown in Figure 10, 13C NMR (101 MHz, C₆D₆) δ 150.9, 147.5, 135.8, 132.7, 123.0, 18.0-16.9 (m, 1C, deuterated positions).

Based on the ¹H NMR data, the deuteration rate was calculated, and the deuteration rate of the carbon-hydrogen bond at the benzyl position on deuterated 3-methylpyridine was determined to be 82%.

### Example 25

### Preparation of Deuterated 1,2-Dimethylindole

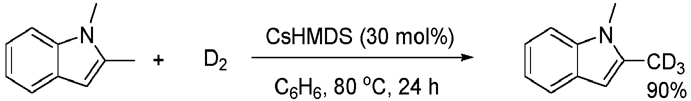

Under an inert atmosphere, in a 25 mL autoclave, 1,2-dimethylindole (CAS: 875-79-6) (0.0436 g, 0.3 mmol), benzene (0.5 mL), and cesium bis(trimethylsilyl)amide (0.0264 g, 0.09 mmol, 30 mol%) were added sequentially. The autoclave was sealed, filled with 4 bar deuterium gas, heated at 80°C for 24 hours to undergo the deuteration reaction. Then, the reaction solution was separated by column chromatography, with a separation yield of 97%. The obtained product was then subjected to ¹H NMR and ¹³C NMR testing using deuterated chloroform (CDCl₃) as the deuterated solvent.

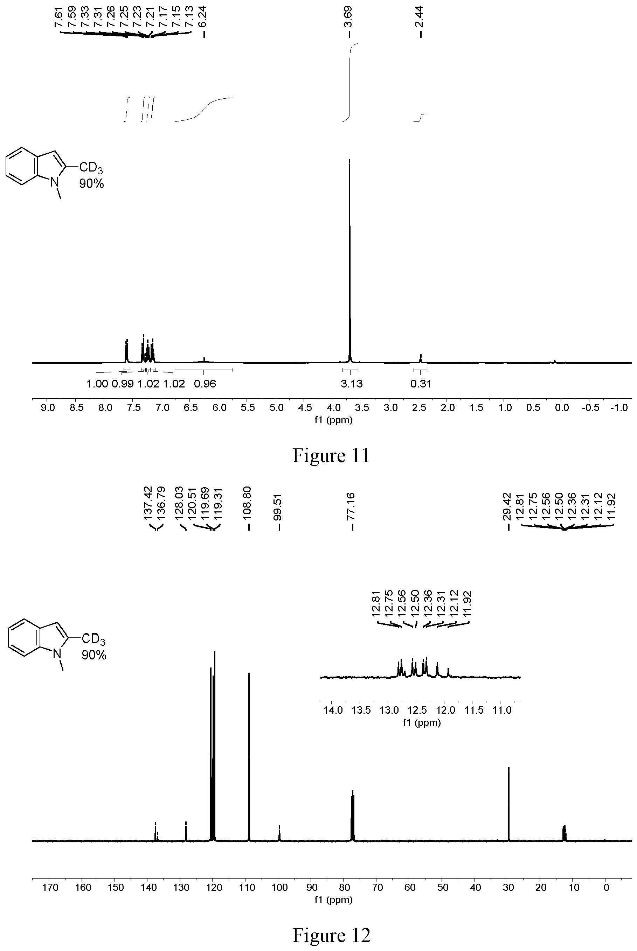

In Example 25, the ¹H NMR spectrum is shown in Figure 11, 1H NMR (400 MHz, CDCl₃) δ 7.65-7.54 (m, 1H), 7.35-7.27 (m, 1H), 7.25-7.19 (m, 1H), 7.18-7.10 (m, 1H), 6.24 (s, 1H), 3.69 (s, 3H), 2.58-2.34 (m, 0.31H, deuterated positions). The ¹³C NMR spectrum is shown in Figure 12, 13C NMR (101 MHz, CDCl₃) δ 137.4, 136.8, 128.0, 120.5, 119.7, 119.3, 108.8, 99.5, 29.42, 12.8-11.9 (m, 1C, deuterated positions).

Based on the ¹H NMR data, the deuteration rate was calculated, and the deuteration rate of the carbon-hydrogen bond at the benzyl positions on deuterated 1,2-dimethylindole was determined to be 90%.

### Example 26

Preparation of Deuterated 2-Methylbenzothiophene

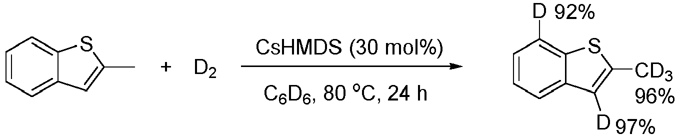

Under an inert atmosphere, in a 25 mL autoclave, 2-methylbenzothiophene (CAS: 1195-14-8) (0.0445 g, 0.3 mmol), deuterated benzene (0.5 mL), and cesium bis(trimethylsilyl)amide (0.0264 g, 0.09 mmol, 30 mol%) were added sequentially. The autoclave was sealed, filled with 4 bar deuterium gas, heated at 80°C for 24 hours to undergo the deuteration reaction. Then, the reaction solution was directly subjected to ¹H NMR and ¹³C NMR testing.

In Example 26, the ¹H NMR spectrum is shown in Figure 13, 1H NMR (400 MHz, C₆D₆) δ 7.60-7.57 (m, 0.08H, deuterated positions), 7.56-7.49 (m, 1H), 7.25-7.15 (m, 1H), 7.13-7.04 (m, 1H), 6.67 (s, 0.03H, deuterated positions), 2.26-2.07 (m, 0.11H, deuterated positions). The ¹³C NMR spectrum is shown in Figure 14, 13C NMR (101 MHz, C₆D₆) δ 141.0, 140.6, 140.2, 124.4, 124.3, 123.8, 123.7, 122.9, 122.3-121.6 (m, 2C, deuterated positions), 15.2-14.4 (m, 1C, deuterated positions).

The deuteration rates were calculated based on ¹H NMR data, the deuteration rates of the carbon-hydrogen bonds at different deuterated positions on deuterated 2-methylbenzothiophene were obtained, as shown in the reaction Equation.

### Example 27

### Preparation of Deuterated Diphenylmethane

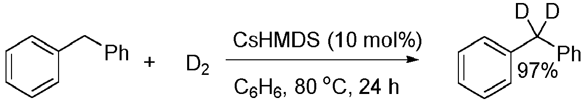

Under an inert atmosphere, in a 25 mL autoclave, diphenylmethane (CAS: 101-81-5) (0.0504 g, 0.3 mmol), benzene (0.5 mL), and cesium bis(trimethylsilyl)amide (0.0087 g, 0.03 mmol, 10 mol%) were added sequentially. The autoclave was sealed, filled with deuterium gas to achieve the pressure of deuterium gas in the autoclave is 4 bar, heated at 80°C for 24 hours to undergo the deuteration reaction. Then, the reaction solution was separated by column chromatography, with a separation yield of 94%. The resulting product was subjected to ¹H NMR and ¹³C NMR testing using deuterated chloroform as the deuterated solvent.

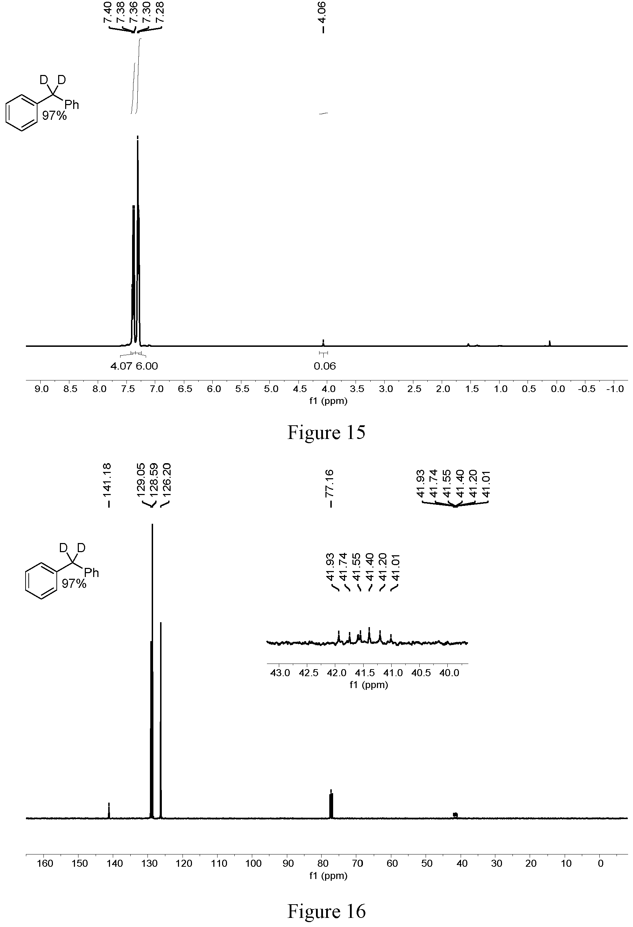

In Example 27, the ¹H NMR spectrum is shown in Figure 15, 1H NMR (400 MHz, CDCl₃) δ 7.43-7.34 (m, 4H), 7.34-7.24 (m, 6H), 4.14-3.99 (m, 0.06H, deuterated positions). The ¹³C NMR spectrum is shown in Figure 16, 13C NMR (101 MHz, CDCl₃) δ 141.2, 129.1, 128.6, 126.2, 41.9-41.0 (m, 1C, deuterated positions).

The deuteration rates were calculated based on ¹H NMR data, the deuteration rate of the carbon-hydrogen bond at the benzyl position on deuterated diphenylmethane was found to be 97%.

### Example 28

### Preparation of Deuterated Benzyl Methyl Ether

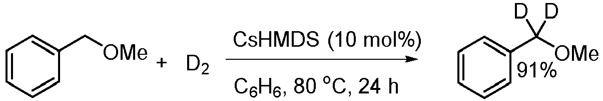

Under an inert atmosphere, in a 25 mL autoclave, benzyl methyl ether (CAS: 538-86-3) (0.0366 g, 0.3 mmol), benzene (0.5 mL), and cesium bis(trimethylsilyl)amide (0.0087 g, 0.03 mmol, 10 mol%) were added sequentially. The autoclave was sealed, filled with deuterium gas to achieve the pressure of deuterium gas in the autoclave is 4 bar, heated at 80°C for 24 hours to undergo the deuteration reaction. Then, the reaction solution was separated by column chromatography, with a separation yield of 42%. The resulting product was subjected to ¹H NMR and ¹³C NMR testing using deuterated chloroform as the deuterated solvent.

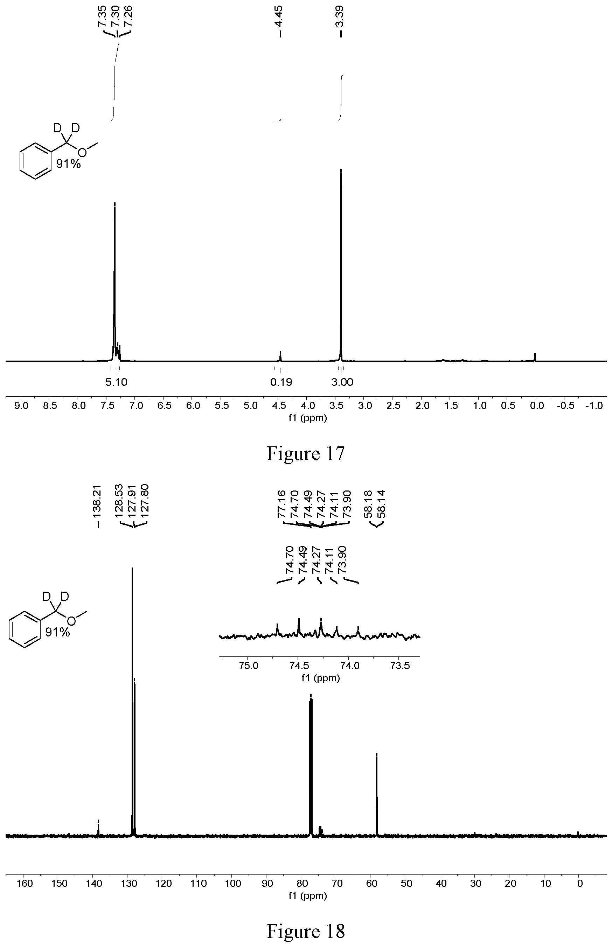

In Example 28, the ¹H NMR spectrum is shown in Figure 17, 1H NMR (400 MHz, CDCl₃) δ 7.42-7.27 (m, 5H), 4.56-4.36 (m, 0.19H, deuterated positions), 3.39 (s, 3H). The ¹³C NMR spectrum is shown in Figure 18, 13C NMR (101 MHz, CDCl₃) δ 138.2, 128.5, 127.9, 127.8, 74.7-73.9 (m, labeled, 1C, deuterated positions), 58.2, 58.1.

The deuteration rates were calculated based on ¹H NMR data, the deuteration rate of the carbon-hydrogen bond at the benzyl position on deuterated benzyl methyl ether was found to be 91%.

### Example 29

### Preparation of Deuterated Benzyl Phenyl Sulfide

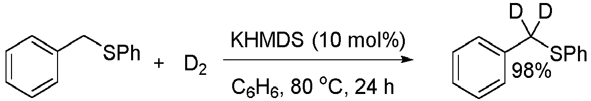

Under an inert atmosphere, in a 25 mL autoclave, benzyl phenyl sulfide (CAS: 831-91-4) (0.0601 g, 0.3 mmol), benzene (0.5 mL), and potassium bis(trimethylsilyl)amide (0.0060 g, 0.03 mmol, 10 mol%) were added sequentially. The autoclave was sealed, filled with deuterium gas to achieve the pressure of deuterium gas in the autoclave is 4 bar, heated at 80°C for 24 hours to undergo the deuteration reaction. Then, the reaction solution was separated by column chromatography, with a separation yield of 99%. The resulting product was subjected to ¹H NMR and ¹³C NMR testing using deuterated chloroform as the deuterated solvent.

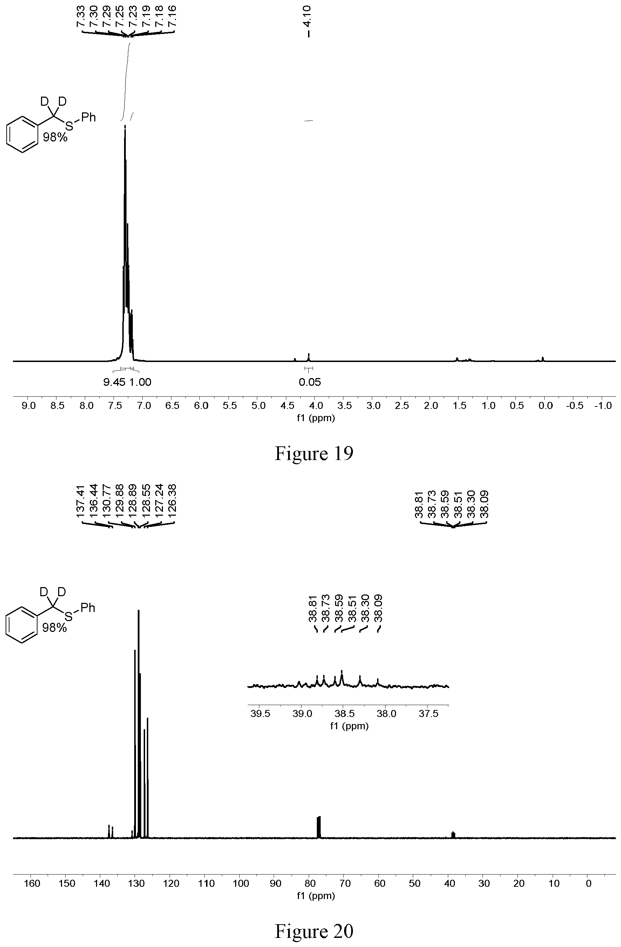

In Example 29, the ¹H NMR spectrum is shown in Figure 19, 1H NMR (400 MHz, CDCl₃) δ 7.37-7.21 (m, 9H), 7.21-7.15 (m, 1H), 4.18-4.03 (m, 0.05H, deuterated positions). The ¹³C NMR spectrum is shown in Figure 20, 13C NMR (101 MHz, CDCl₃) δ 137.4, 136.4, 130.8, 129.9, 128.9, 128.6, 127.2, 126.4, 38.8-38.1 (m, 1C, deuterated positions).

The deuteration rates were calculated based on ¹H NMR data, the deuteration rate of the carbon-hydrogen bond at the benzyl position on deuterated benzyl phenyl sulfide was found to be 98%.

### Example 30

### Preparation of Deuterated N-Ethyl-N-Benzyl Aniline

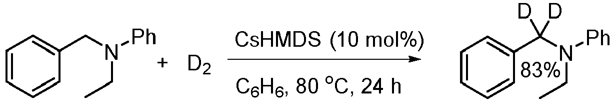

Under an inert atmosphere, in a 25 mL autoclave, N-ethyl-N-benzyl aniline (CAS: 92-59-1) (0.0634 g, 0.3 mmol), benzene (0.5 mL), and cesium bis(trimethylsilyl)amide (0.0087 g, 0.03 mmol, 10 mol%) were added sequentially. The autoclave was sealed, filled with deuterium gas to achieve the pressure of deuterium gas in the autoclave is 4 bar, heated at 80°C for 24 hours to undergo the deuteration reaction. Then, the reaction solution was subjected to column chromatography, with a separation yield of 98%. The resulting product was subjected to ¹H NMR and ¹³C NMR testing using deuterated chloroform as the deuterated solvent.

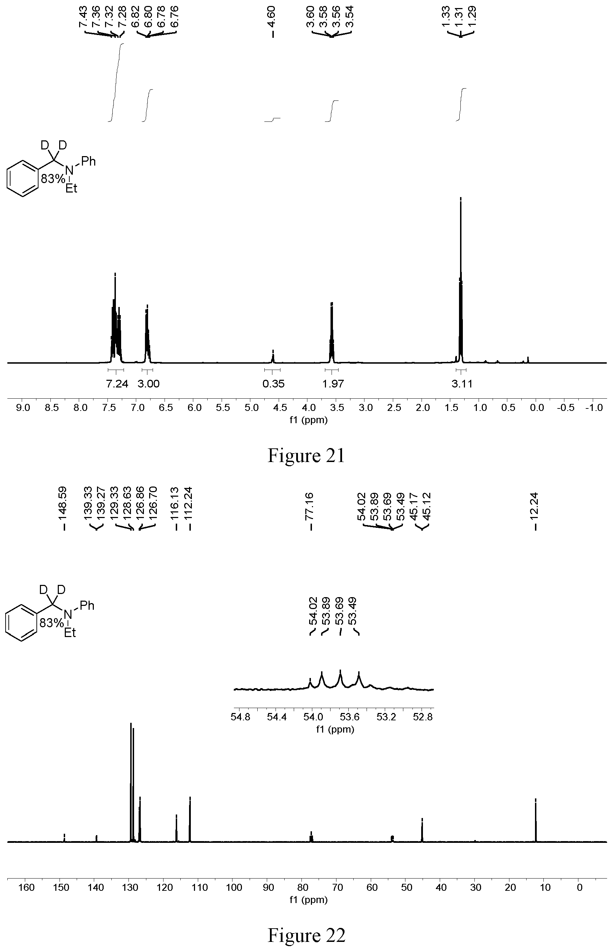

In Example 30, the ¹H NMR spectrum is shown in Figure 21, 1H NMR (400 MHz, CDCl₃) δ 7.49-7.21 (m, 7H), 6.90-6.71 (m, 3H), 4.75-4.47 (m, 0.35H, deuterated positions), 3.69-3.45 (m, 2H), 1.31 (t, *J* = 7.0 Hz, 3H). The ¹³C NMR spectrum is shown in Figure 22, 13C NMR (101 MHz, CDCl₃) δ 148.6, 139.33, 139.27, 129.3, 128.6, 126.9, 126.7, 116.1, 112.2, 54.0-53.5 (m, 1C, deuterated positions), 45.2, 45.1, 12.2.

The deuteration rates were calculated based on ¹H NMR data, the deuteration rate of the carbon-hydrogen bond at the benzyl position on deuterated N-ethyl-N-benzyl aniline was found to be 83%.

### Example 31

### Preparation of Deuterated Phenyl Methyl Sulfide

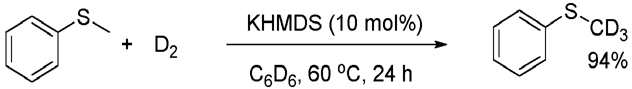

Under an inert atmosphere, in a 25 mL autoclave, phenyl methyl sulfide (CAS: 100-68-5) (0.0372 g, 0.3 mmol), deuterated benzene (0.5 mL), and potassium bis(trimethylsilyl)amide (0.0060 g, 0.03 mmol, 10 mol%) were added sequentially. The autoclave was sealed, filled with deuterium gas to achieve the pressure of deuterium gas in the autoclave is 4 bar, heated at 60 °C for 24 hours to undergo the deuteration reaction. Then, the reaction solution was subjected to ¹H NMR and ¹³C NMR testing directly.

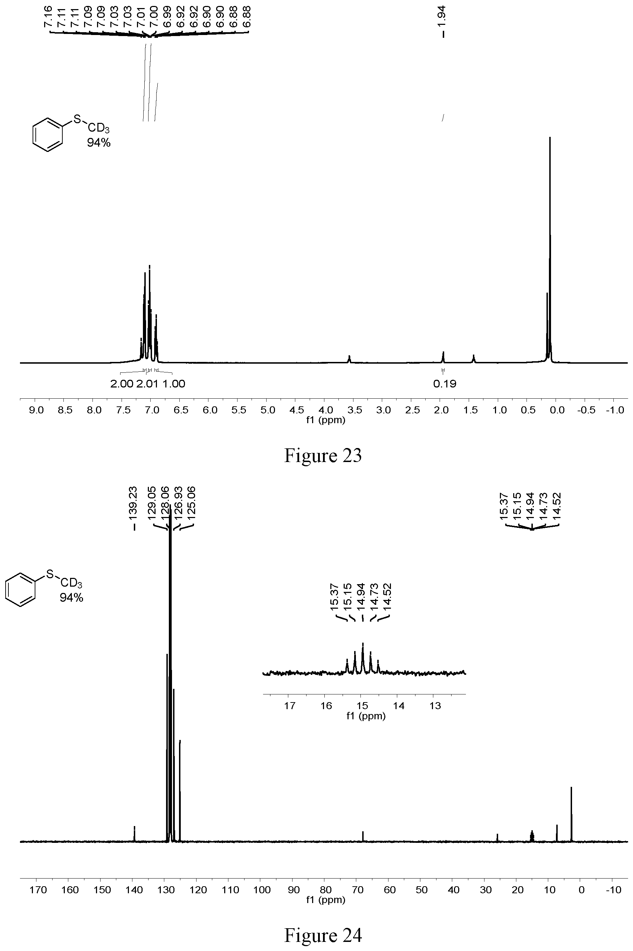

In Example 31, the ¹H NMR spectrum is shown in Figure 23, 1H NMR (400 MHz, C₆D₆) δ 7.13-7.08 (m, 2H), 7.04-6.99 (m, 2H), 6.93-6.87 (m, 1H), 1.97-1.93 (m, 0.19H, deuterated positions). The ¹³C NMR spectrum is shown in Figure 24, 13C NMR (101 MHz, C₆D₆) δ 139.2, 129.1, 126.9, 125.1, 15.4-14.5 (m, 1C, deuterated positions).

The deuteration rates were calculated based on ¹H NMR data, the deuteration rate of the carbon-hydrogen bond at the deuterated position in deuterated phenyl methyl sulfide was found to be 94%.

### Example 32

### Preparation of Deuterated Methylthiocyclohexyl Ester

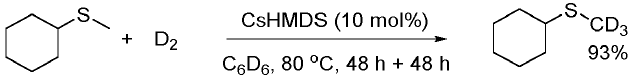

Under an inert atmosphere, in a 25 mL autoclave, methylthiocyclohexyl ester (CAS: 7133-37-1) (0.0391 g, 0.3 mmol), deuterated benzene (0.5 mL), and cesium bis(trimethylsilyl)amide (0.0087 g, 0.03 mmol, 10 mol%) were added sequentially. The autoclave was sealed, filled with 4 bar deuterium gas, heated at 80°C for 48 hours to undergo the deuteration reaction. Then, deuterium gas was refilled to maintain a pressure of 4 bar for the deuterium gas inside the autoclave, and the reaction was continued for another 48 hours. Afterward, the reaction solution was subjected to ¹H NMR and ¹³C NMR testing, and the deuteration rate is shown in the figure.

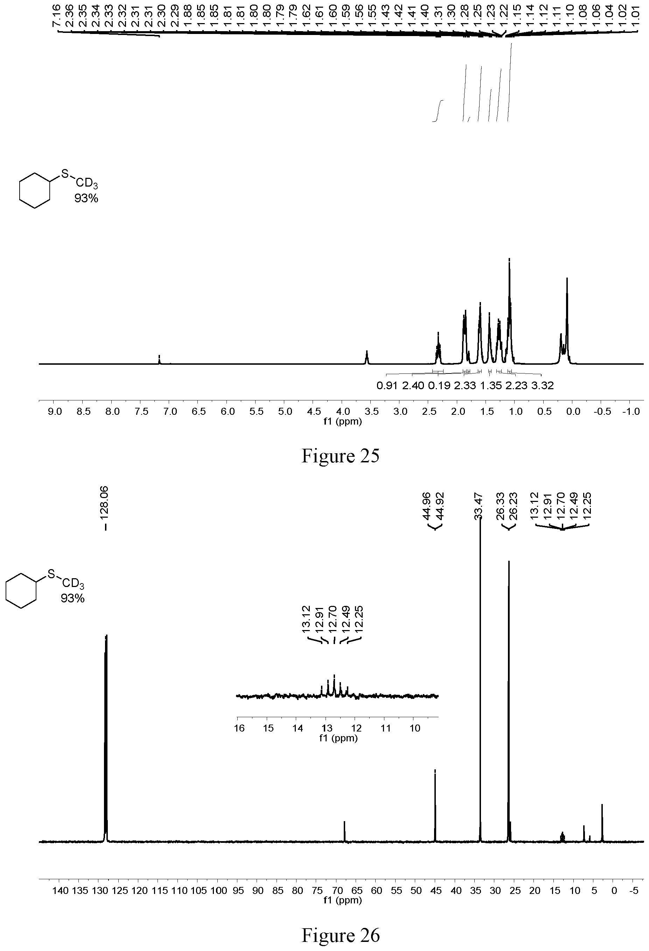

In Example 32, the ¹H NMR spectrum is shown in Figure 25, 1H NMR (400 MHz, C₆D₆) δ 2.42-2.24 (m, 1H), 1.90-1.83 (m, 2H), 1.82-1.77 (m, 0.19H, deuterated positions), 1.64-1.57 (m, 2H), 1.45-1.40 (m, 1H), 1.31-1.23 (m, 2H), 1.12-1.05 (m, 3H). The ¹³C NMR spectrum is shown in Figure 26, 13C NMR (101 MHz, C₆D₆) δ 45.0, 44.9, 33.5, 26.3, 26.2, 13.1-12.3 (m, 1C, deuterated positions).

The deuteration rates were calculated based on ¹H NMR data, the deuteration rate of the carbon-hydrogen bond at the deuterated position in deuterated methylthiocyclohexyl ester was found to be 93%.

### Example 33

### Preparation of Deuterated Dimethyl Sulfoxide

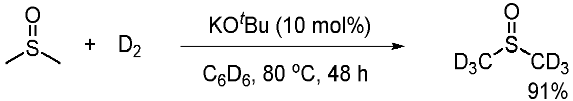

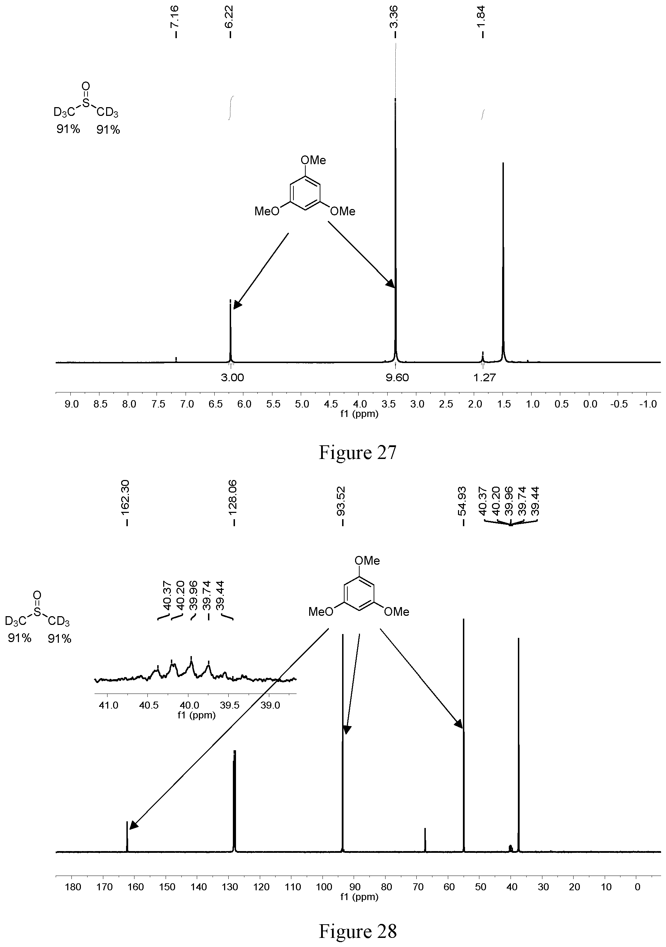

Under an inert atmosphere, in a 25 mL autoclave, dimethyl sulfoxide (CAS: 67-68-5) (0.0234 g, 0.3 mmol), deuterated benzene (0.5 mL), and potassium tert-butoxide (0.0034 g, 0.03 mmol, 10 mol%) were added sequentially. The autoclave was sealed, filled with 4 bar deuterium gas, heated at 80°C for 48 hours to undergo the deuteration reaction. Subsequently, the reaction solution was treated with 1,3,5-Trimethoxybenzene (0.0218 g) as an internal standard, and then subjected to ¹H NMR and ¹³C NMR testing. The ¹H NMR spectrum is shown in Figure 27, and the ¹³C NMR spectrum is shown in Figure 28.

The deuteration rates were calculated based on ¹H NMR data, the deuteration rate of the the carbon-hydrogen bond at the deuterated position in deuterated dimethyl sulfoxide was found to be 91%.

### Example 34

### Preparation of Deuterated Phenyl Methyl Sulfoxide

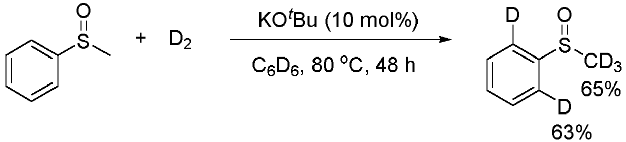

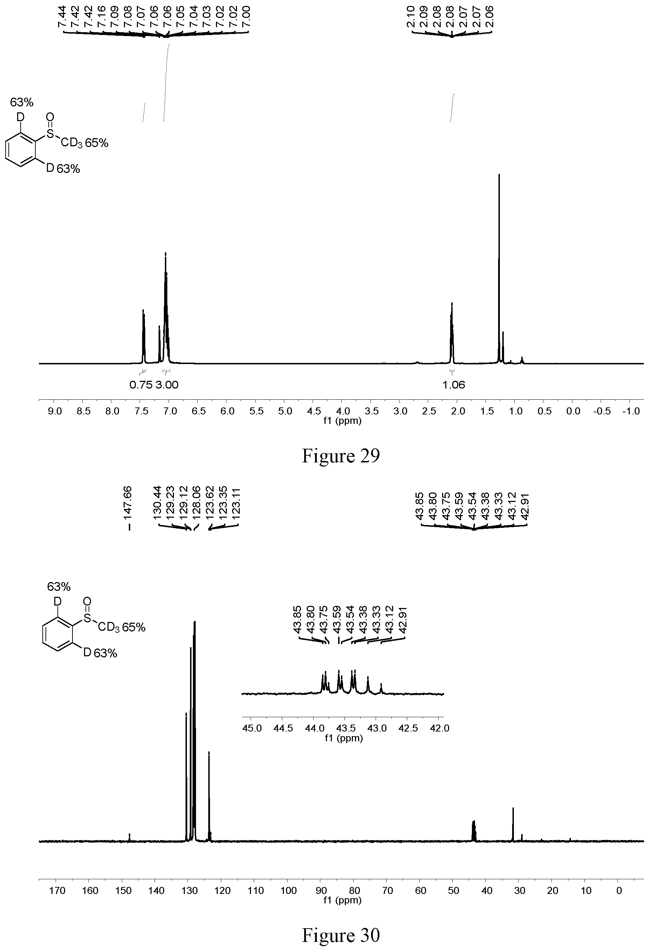

Under an inert atmosphere, in a 25 mL autoclave, phenyl methyl sulfoxide (CAS: 1193-82-4) (0.0421 g, 0.3 mmol), deuterated benzene (0.5 mL), and potassium tert-butoxide (0.0034 g, 0.03 mmol, 10 mol%) were added sequentially. The autoclave was sealed, filled with 4 bar deuterium gas, heated at 80°C for 48 hours to undergo the deuteration reaction. Subsequently, the reaction solution was subjected to ¹H NMR and ¹³C NMR testing directly. The ¹H NMR spectrum is shown in Figure 29, and the ¹³C NMR spectrum is shown in Figure 30.

The deuteration rates were calculated based on ¹H NMR data, to obtain the deuteration rates of the C-H bonds at different deuterated positions in deuterated phenyl methyl sulfoxide, as shown in the reaction equation.

### Example 35

### Preparation of Deuterated N,N-dimethylmethanesulfonamide

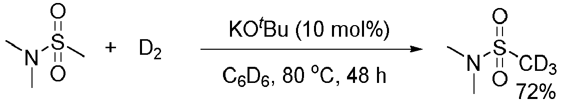

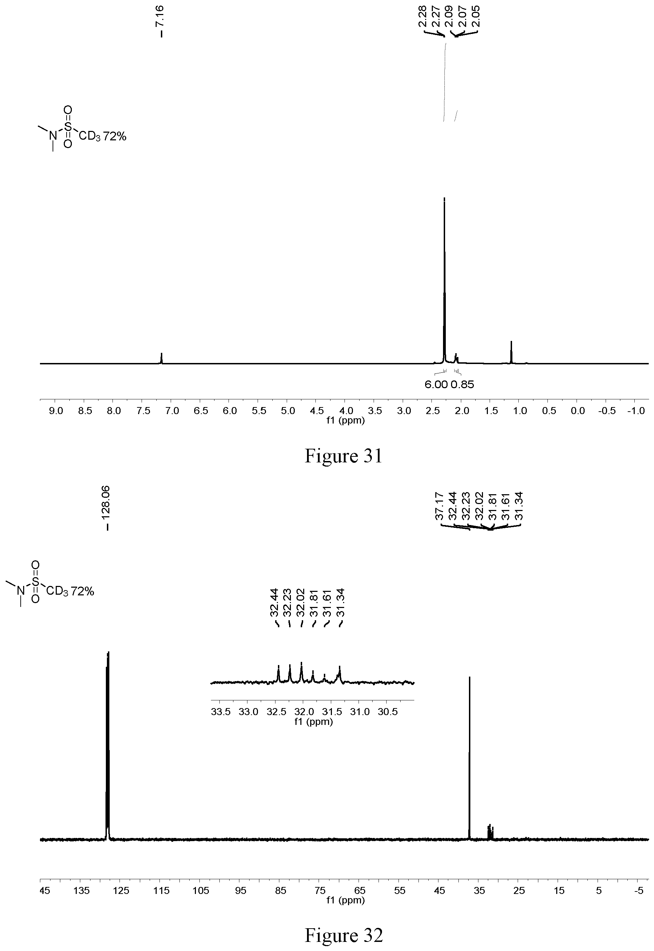

Under an inert atmosphere, in a 25 mL autoclave, *N,N-*dimethylmethanesulfonamide (CAS: 918-05-8) (0.0370 g, 0.3 mmol), deuterated benzene (0.5 mL), and potassium tert-butoxide (0.0034 g, 0.03 mmol, 10 mol%) were added sequentially. The autoclave was sealed, filled with 4 bar deuterium gas, heated at 80°C for 48 hours to undergo the deuteration reaction. Subsequently, the reaction solution was subjected to ¹H NMR and ¹³C NMR testing directly. The ¹H NMR spectrum is shown in Figure 31, and the ¹³C NMR spectrum is shown in Figure 32.

The deuteration rate was calculated based on ¹H NMR data, the deuteration rate of the carbon-hydrogen bond at the deuterated position in deuterated *N,N-*dimethylmethanesulfonamide was found to be 72%.

### Example 36

### Preparation of Deuterated Anisole

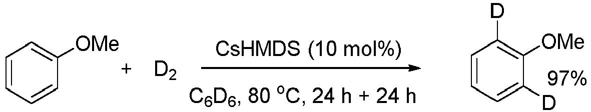

Under an inert atmosphere, in a 25 mL autoclave, anisole (CAS: 100-66-3) (0.0324 g, 0.3 mmol), deuterated benzene (0.5 mL), and cesium bis(trimethylsilyl)amide (0.0087 g, 0.03 mmol, 10 mol%) were added sequentially. The autoclave was sealed, filled with 4 bar deuterium gas, heated at 80°C for 24 hours to undergo the deuteration reaction. Deuterium gas was refilled to maintain a pressure of 4 bar for the deuterium gas inside the autoclave, and the reaction was continued for another 24 hours. Subsequently, the reaction solution was subjected to ¹H NMR and ¹³C NMR analysis directly.

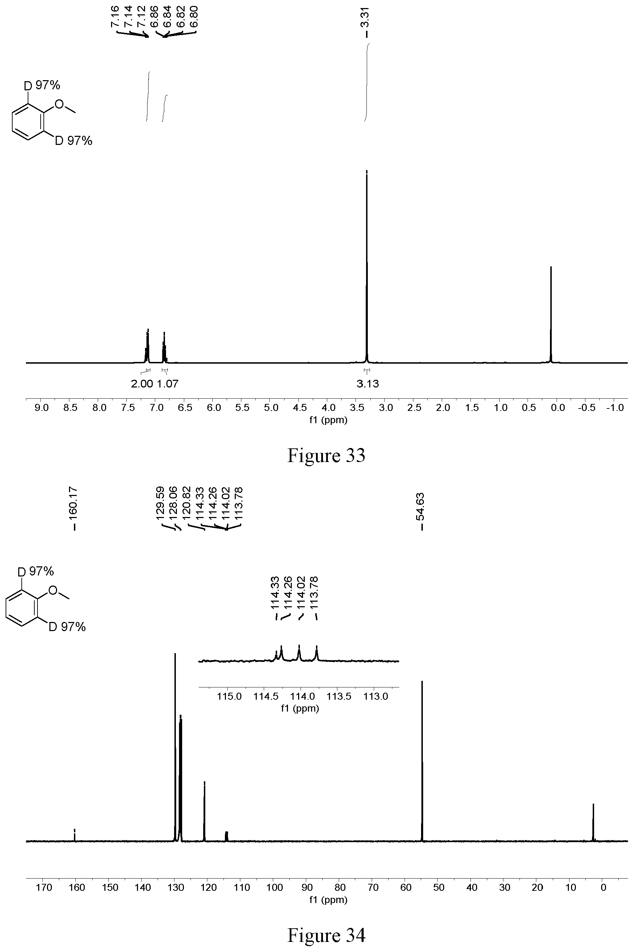

In Example 36, ¹H NMR hydrogen spectrum is shown in Figure 33, 1H NMR (400 MHz, C₆D₆) δ 7.15-7.09 (m, 2H), 6.88-6.78 (m, 1.07H, deuterated positions), 3.31 (s, 3H). The ¹³C NMR carbon spectrum is shown in Figure 34, 13C NMR (101 MHz, C₆D₆) δ 160.2, 129.6, 120.8, 114.3-113.8 (m, 2C, deuterated positions), 54.6.

The deuteration rate was calculated based on ¹H NMR data, the deuteration rate of the carbon-hydrogen bond at the deuterated position in deuterated anisole was found to be 97%.

### Example 37

### Preparation of Deuterated 1,3-Benzodioxole

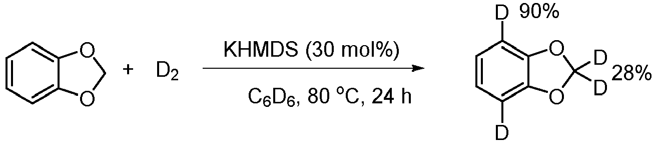

Under an inert atmosphere, in a 25 mL autoclave, 1,3-benzodioxole (CAS: 274-09-9) (0.0367 g, 0.3 mmol), deuterated benzene (0.5 mL), and potassium bis(trimethylsilyl)amide (0.0180 g, 0.09 mmol, 30 mol%) were added sequentially. The autoclave was sealed, filled with 4 bar deuterium gas, heated at 80°C for 24 hours to undergo the deuteration reaction. Subsequently, the reaction solution was subjected to ¹H NMR and ¹³C NMR testing directly.

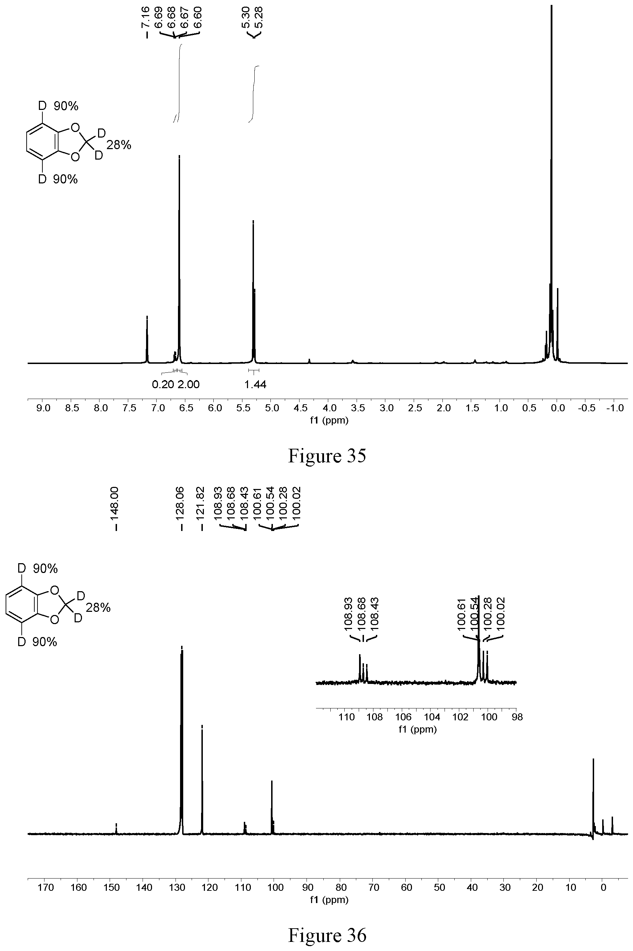

In Example 37, ¹H NMR hydrogen spectrum is shown in Figure 35, 1H NMR (400 MHz, C₆D₆) δ 6.71-6.64 (m, 0.20H, deuterated positions), 6.60 (s, 2H), 5.39-5.20 (m, 1.44H, deuterated positions). The ¹³C NMR carbon spectrum is shown in Figure 36, 13C NMR (101 MHz, C₆D₆) δ 148.0, 121.8, 108.9-108.4 (m, 2C, deuterated positions), 100.6-100.0 (m, 1C, deuterated positions).

The deuteration rates were calculated based on ¹H NMR data to obtain the deuteration rates of the C-H bonds at different deuterated positions in deuterated 1,3-benzodioxole, as shown in the reaction equation.

### Example 38

### Preparation of Deuterated 4-Fluorobiphenyl

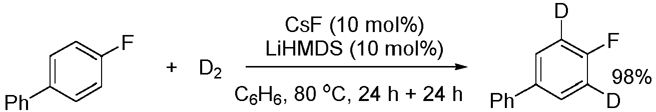

Under an inert atmosphere, in a 25 mL autoclave, 4-fluorobiphenyl (CAS: 324-74-3) (0.0516 g, 0.3 mmol), benzene (0.5 mL), cesium fluoride (0.0046 g, 0.03 mmol, 10 mol%), and lithium bis(trimethylsilyl)amide (0.0050 g, 0.03 mmol, 10 mol%) were added sequentially. The autoclave was sealed, filled with 4 bar deuterium gas, heated at 80°C for 24 hours to undergo the deuteration reaction. Subsequently, deuterium gas was refilled to maintain a pressure of 4 bar for the deuterium gas inside the autoclave, and the reaction was continued for another 24 hours. Then, the reaction solution was separated by column chromatography, with a separation yield of 98%. The obtained product was analyzed by ¹H NMR and ¹³C NMR, using deuterated chloroform as the deuterated solvent.

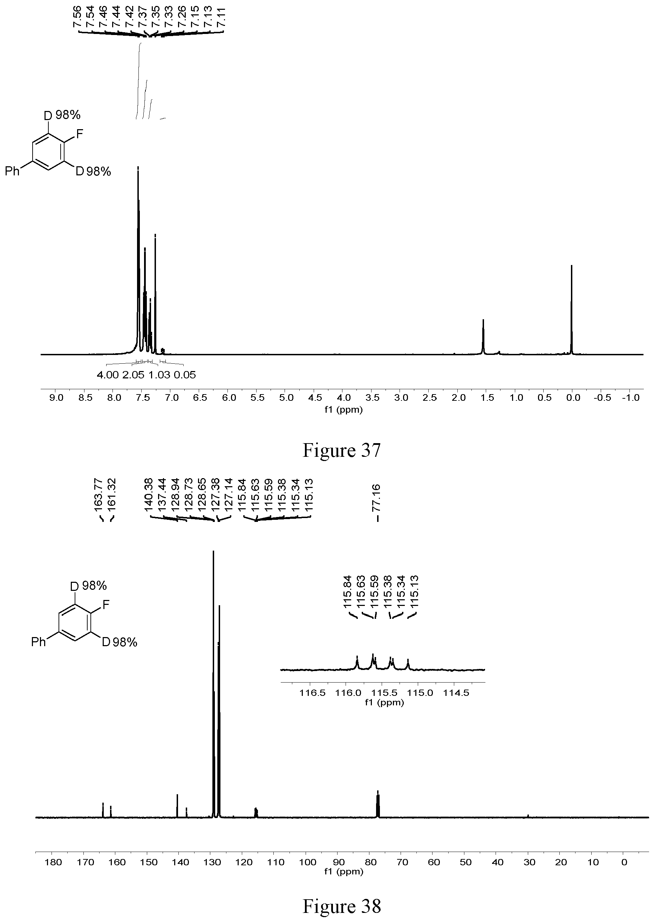

In Example 38, ¹H NMR hydrogen spectrum is shown in Figure 37, 1H NMR (400 MHz, CDCl₃) δ 7.59-7.50 (m, 4H), 7.48-7.40 (m, 2H), 7.38-7.31 (m, 1H), 7.18-7.08 (m, 0.05H, deuterated positions). The ¹³C NMR carbon spectrum is shown in Figure 38, 13C NMR (101 MHz, CDCl₃) δ 162.6 (d, *J* = 246.4Hz), 140.4, 137.4, 128.9, 128.7 (d, *J* = 8.0Hz), 127.4, 127.1, 115.8-115.1 (m, 2C, deuterated positions).

The deuteration rate was calculated based on ¹H NMR data, the deuteration rate of the carbon-hydrogen bond at the deuterated position in deuterated 4-fluorobiphenyl was found to be 98%.

### Example 39

### Preparation of Deuterated 1,4-Difluorobenzene

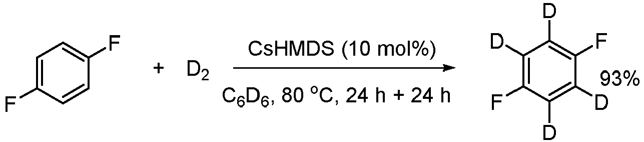

Under an inert atmosphere, in a 25 mL autoclave, 1,4-difluorobenzene (CAS: 540-36-3) (0.0342 g, 0.3 mmol), deuterated benzene (0.5 mL), and cesium bis(trimethylsilyl)amide (0.0087 g, 0.03 mmol, 10 mol%) were added sequentially. The autoclave was sealed, filled with 4 bar deuterium gas, heated at 80°C for 24 hours to undergo the deuteration reaction. Subsequently, deuterium gas was refilled to maintain a pressure of 4 bar for the deuterium gas inside the autoclave, and the reaction was continued for another 24 hours. Then, dibromomethane (CAS: 74-95-3) (0.0264 g) was added to the reaction solution as an internal standard. The reaction solution was directly subjected to ¹H NMR and ¹³C NMR testing.

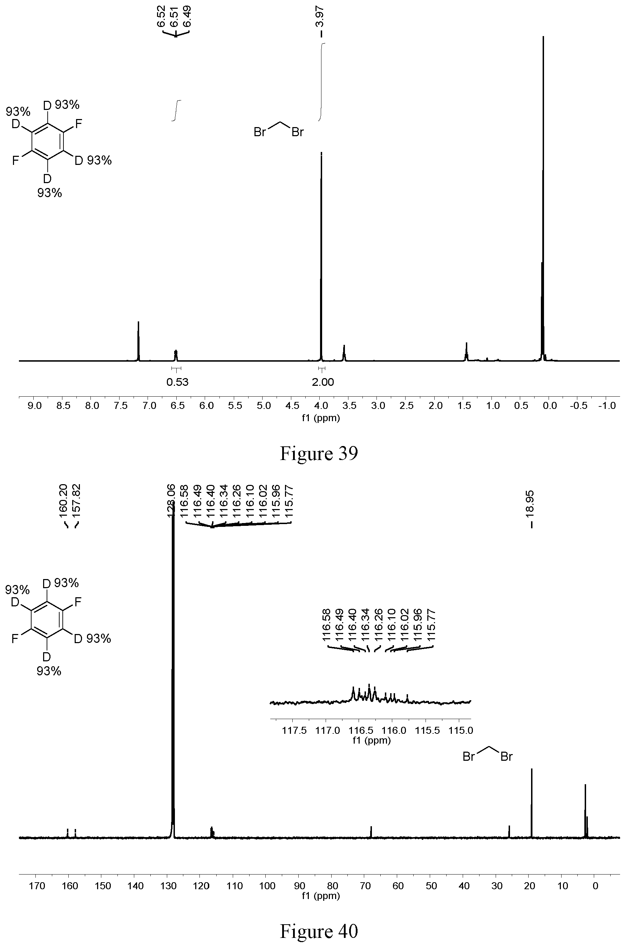

In Example 39, ¹H NMR hydrogen spectrum is shown in Figure 39, 1H NMR (400 MHz, C₆D₆) δ 6.58-6.42 (m, 0.53H, deuterated positions), 3.97 (s, 2H, internal standard dibromomethane). the ¹³C NMR carbon spectrum is shown in Figure 40, 13C NMR (101 MHz, C₆D₆) δ 159.0 (d, J= 239.3Hz), 116.6-115.8 (m, 4C, deuterated positions), 19.0 (Internal standard dibromomethane).

The deuteration rate was calculated based on ¹H NMR data, the deuteration rate of the carbon-hydrogen bond at the deuterated position in deuterated 1,4-difluorobenzene was found to be 93%.

### Example 40

### Preparation of Deuterated Benzene

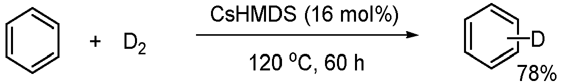

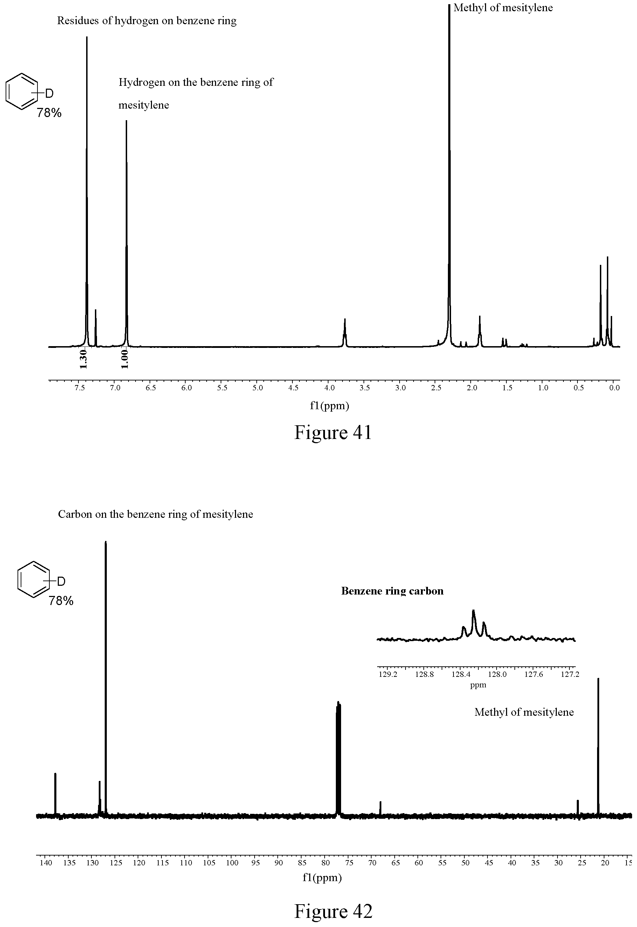

Under an inert atmosphere, in a 25 mL autoclave, benzene (CAS: 71-43-2) (0.5000 g, 6.4 mmol) and cesium bis(trimethylsilyl)amide (0.3000 g, 1.02 mmol, 16 mol%) were added sequentially. The autoclave was sealed, filled with 14 bar deuterium gas, heated at 120°C for 60 hours to undergo the deuteration reaction. Subsequently, the reaction solution was separated and purified, added with mesitylene (2.1 mmol) as an internal standard, then subjected to ¹H NMR and ¹³C NMR testing. ¹H NMR hydrogen spectrum is shown in Figure 41, the ¹³C NMR carbon spectrum is shown in Figure 42.

The deuteration rate was calculated based on ¹H NMR data, the deuteration rate of the carbon-hydrogen bond at the deuterated position in deuterated benzene was found to be 78%.

### Example 41

### Preparation of Deuterated Biphenyl

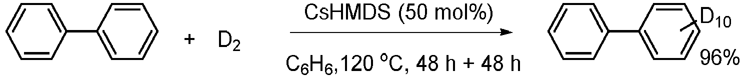

Under an inert atmosphere, in a 25 mL autoclave, biphenyl (CAS: 92-52-4) (0.0463 g, 0.3 mmol), benzene (0.5 mL) and cesium bis(trimethylsilyl)amide (0.0435 g, 0.15 mmol, 50 mol%) were added sequentially. The autoclave was sealed, filled with 4 bar deuterium gas, heated at 120°C for 48 hours to undergo the deuteration reaction. Deuterium gas was refilled to maintain a pressure of 4 bar for the deuterium gas inside the autoclave, and the reaction was continued for another 48 hours. The average deuteration rate was determined to be 96% through the analysis using Gas Chromatography-Mass Spectrometry (GC-MS) and IsoPat² software.

### Example 42

### Preparation of Deuterated Naphthalene

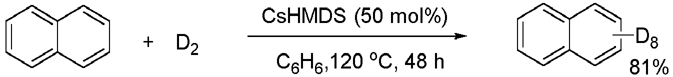

Under an inert atmosphere, in a 25 mL autoclave, naphthalene (CAS: 91-20-3) (0.0385 g, 0.3 mmol) benzene (0.5 mL) and cesium bis(trimethylsilyl)amide (0.0435 g, 0.15 mmol, 50 mol%) were added sequentially. The autoclave was sealed, filled with 4 bar deuterium gas, heated at 120°C for 48 hours to undergo the deuteration reaction. The average deuteration rate was determined to be 81% through the analysis using Gas Chromatography-Mass Spectrometry (GC-MS) and IsoPat² software .

### Example 43

### Preparation of Deuterated Triphenylamine

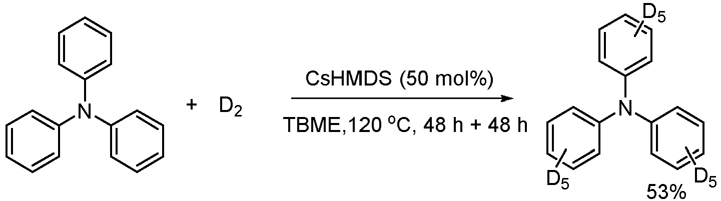

Under an inert atmosphere, in a 25 mL autoclave, triphenylamine (CAS: 603-34-9) (0.0736 g, 0.3 mmol), methyl tert-butyl ether (1.0 mL) and cesium bis(trimethylsilyl)amide (0.0435 g, 0.15 mmol, 50 mol%) were added sequentially. The autoclave was sealed, filled with 4 bar deuterium gas, heated at 120°C for 48 hours to undergo the deuteration reaction. Deuterium gas was refilled to maintain a pressure of 4 bar for the deuterium gas inside the autoclave, and the reaction continued for another 48 hours. The average deuteration rate was determined to be 53% through the analysis using Gas Chromatography-Mass Spectrometry (GC-MS) and IsoPat² software.

### Example 44

### Preparation of Deuterated N-Phenylcarbazole

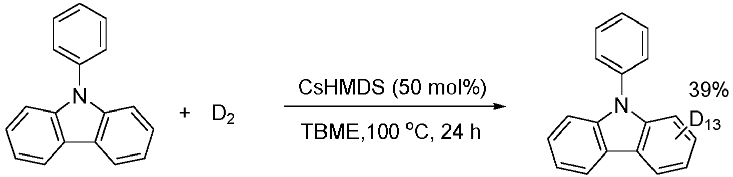

Under an inert atmosphere, in a 25 mL autoclave, N-phenylcarbazole (CAS: 1150-62-5) (0.0730 g, 0.3 mmol), methyl tert-butyl ether (0.5 mL) and cesium bis(trimethylsilyl)amide (0.0435 g, 0.15 mmol, 50 mol%) were added sequentially. The autoclave was sealed, filled with 4 bar deuterium gas, heated at 100°C for 24 hours to undergo the deuteration reaction. The average deuteration rate was determined to be 39% through the analysis using Gas Chromatography-Mass Spectrometry (GC-MS) and IsoPat² software.

### Example 45

### Preparation of Deuterated Pyridine

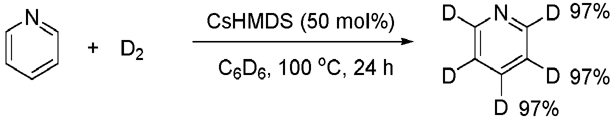

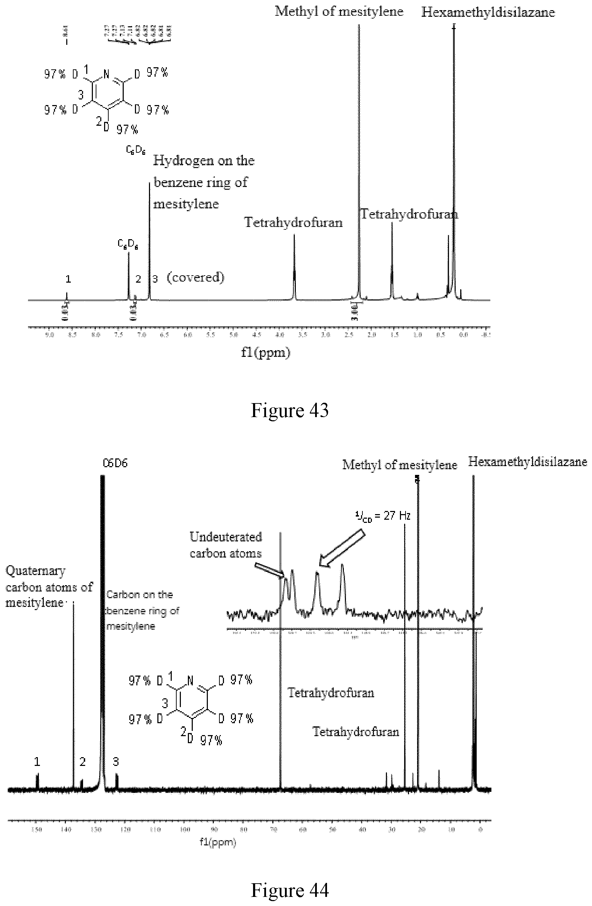

Under an inert atmosphere, in a 25 mL autoclave, pyridine (CAS: 110-86-1) (0.0237 g, 0.3 mmol), deuterated benzene (0.5 mL) and cesium bis(trimethylsilyl)amide (0.0435 g, 0.15 mmol, 50 mol%) were added sequentially. The autoclave was sealed, filled with 4 bar deuterium gas, heated at 100°C for 24 hours to undergo the deuteration reaction. Then, the reaction solution was added with mesitylene (0.0120 g, 0.1 mmol) as an internal standard, and subjected to ¹H NMR and ¹³C NMR testing. The ¹H NMR spectrum is shown in Figure 43, and the ¹³C NMR spectrum is shown in Figure 44.

The deuteration rate was calculated based on ¹H NMR data, the deuteration rate of the carbon-hydrogen bond at the deuterated position in deuterated pyridine was found to be 97%.

### Example 46

### Preparation of Deuterated 2-Phenylpyridine

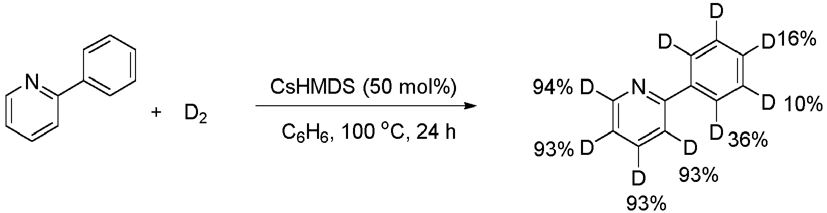

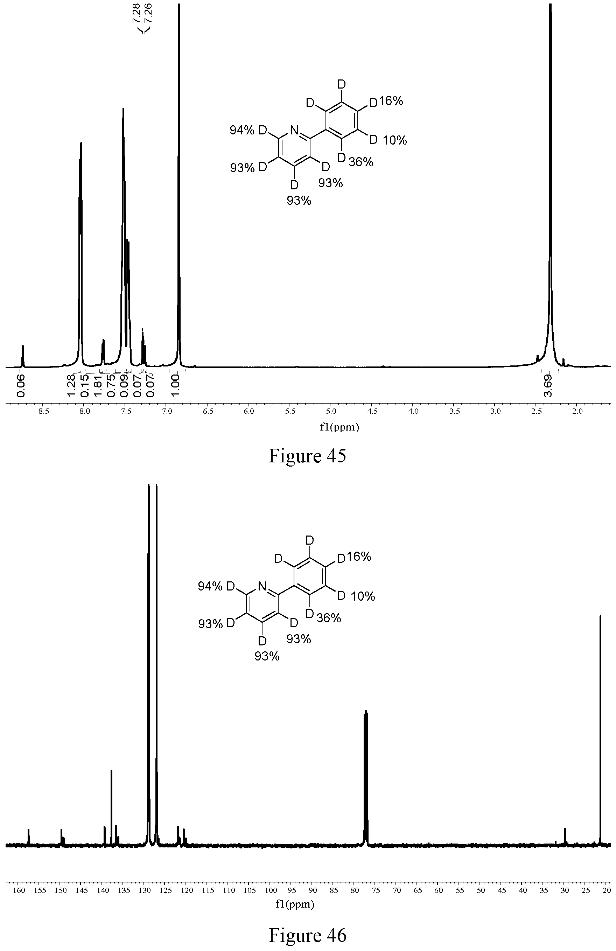

Under an inert atmosphere, in a 25 mL autoclave, 2-phenylpyridine (CAS: 1008-89-5) (0.0467 g, 0.3 mmol), benzene (0.5 mL) and cesium bis(trimethylsilyl)amide (0.0435 g, 0.15 mmol, 50 mol%) were added sequentially. The autoclave was sealed, filled with 4 bar deuterium gas, heated at 100°C for 24 hours to undergo the deuteration reaction. Then, the reaction solution was separated and purified, added with mesitylene (0.0120 g, 0.1 mmol) as an internal standard, subjected to ¹H NMR and ¹³C NMR testing. The ¹H NMR spectrum is shown in Figure 45, and the ¹³C NMR spectrum is shown in Figure 46.

The deuteration rates were calculated based on ¹H NMR data, the deuteration rates of the carbon-hydrogen bonds at different positions in deuterated 2-phenylpyridine were obtained as shown in the reaction equation.

### Example 47

### Preparation of Deuterated 2,2'-Bipyridine

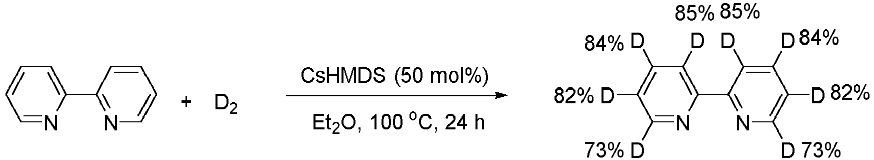

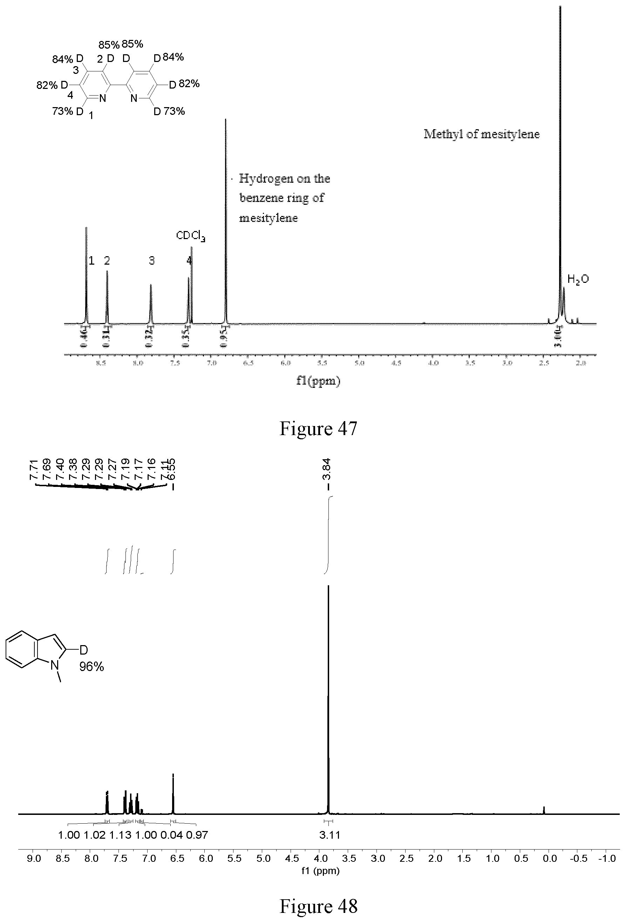

Under an inert atmosphere, in a 25 mL autoclave, 2,2'-bipyridine (CAS: 366-18-7) (0.0468 g, 0.3 mmol), ether (0.5 mL) and cesium bis(trimethylsilyl)amide (0.0435 g, 0.15 mmol, 50 mol%) were added sequentially. The autoclave was sealed, filled with 4 bar deuterium gas, heated at 100°C for 24 hours to undergo the deuteration reaction. Then, the reaction solution was separated and purified, and added with mesitylene (0.0120 g, 0.1 mmol) as an internal standard, subjected to ¹H NMR and ¹³C NMR testing. The ¹H NMR spectrum is shown in Figure 47.

The deuteration rates were calculated based on ¹H NMR data, the deuteration rates of the carbon-hydrogen bonds at different positions in deuterated 2,2'-bipyridine were obtained as shown in the reaction equation.

### Example 48

### Preparation of Deuterated N-Methylindole

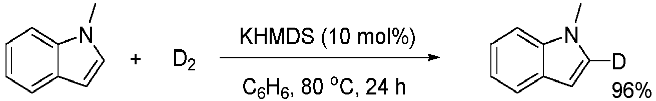

Under an inert atmosphere, in a 25 mL autoclave, N-methylindole (CAS: 603-76-9) (0.0394 g, 0.3 mmol), benzene (0.5 mL) and potassium bis(trimethylsilyl)amide (0.0060 g, 0.03 mmol, 10 mol%) were added sequentially. The autoclave was sealed, filled with 4 bar deuterium gas, heated at 80°C for 24 hours to undergo the deuteration reaction. Then, the reaction solution was separated by column chromatography with a separation yield of 95%. The obtained product was subjected to ¹H NMR and ¹³C NMR testing, using deuterated chloroform (CDCl₃) as the deuterated solvent.

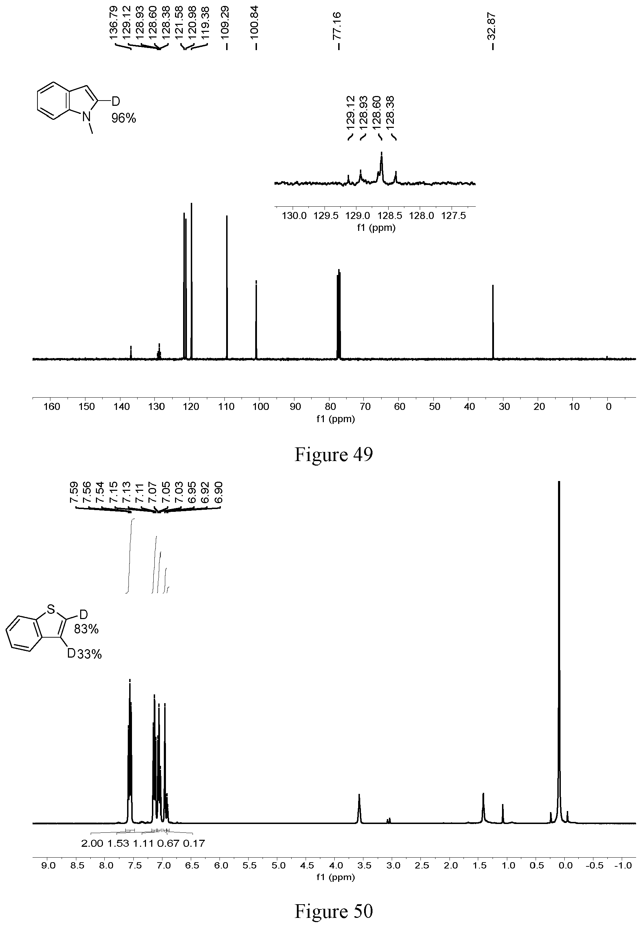

In Example 47, ¹H NMR hydrogen spectrum is shown in Figure 48, 1H NMR (400 MHz, CDCl₃) δ 7.74-7.66 (m, 1H), 7.42-7.35 (m, 1H), 7.33-7.25 (m, 1H), 7.21-7.14 (m, 1H), 7.13-7.07 (m, 0.04H, deuterated positions), 6.55 (s, 1H), 3.84 (s, 3H). The ¹³C NMR carbon spectrum is shown in Figure 49, 13C NMR (101 MHz, CDCl₃) δ 136.8, 128.6, 129.1-128.4 (m, 1C, deuterated positions), 121.6, 121.0, 119.4, 109.3, 101.8, 32.9.

The deuteration rate was calculated based on ¹H NMR data, the deuteration rate of the carbon-hydrogen bond at the deuterated position in deuterated N-methylindole was found to be 96%.

### Example 49

### Preparation of Deuterated Benzothiophene

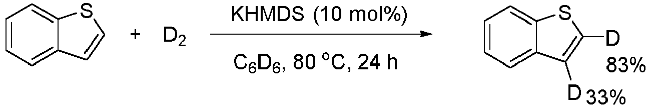

Under an inert atmosphere, in a 25 mL autoclave, benzothiophene (CAS: 95-15-8) (0.0403 g, 0.3 mmol), deuterated benzene (0.5 mL) and potassium bis(trimethylsilyl)amide (0.0060 g, 0.03 mmol, 10 mol%) were added sequentially. The autoclave was sealed, filled with 4 bar deuterium gas, heated at 80°C for 24 hours to undergo the deuteration reaction. Then, the reaction solution was directly subjected to ¹H NMR and ¹³C NMR testing.

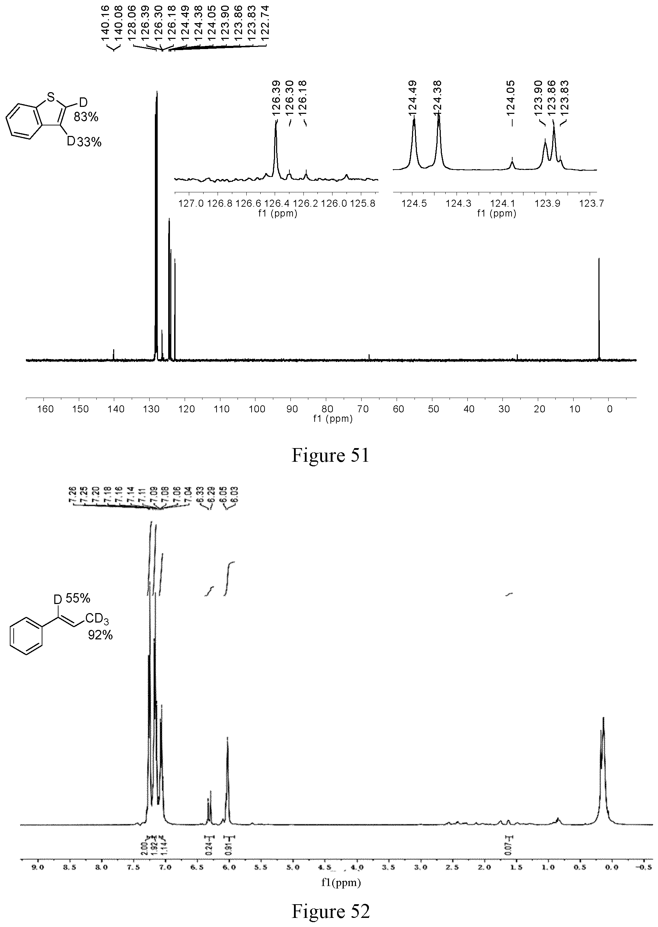

In Example 49, the ¹H NMR spectrum is shown in Figure 50, 1H NMR (400 MHz, C₆D₆) δ 7.64-7.48 (m, 2H), 7.18-7.09 (m, 1H), 7.05 (t, J = 7.6Hz, 1H), 6.99-6.93 (m, 0.67H, deuterated positions), 6.92-6.88 (m, 0.17H, deuterated positions). The ¹³C NMR spectrum is shown in Figure 51, 13C NMR (101 MHz, C₆D₆) δ 140.2, 140.1, 126.4-126.2 (m, 1C, deuterated positions), 124.5, 124.4, 124.0 (1C, deuterated positions), 123.90, 123.86, 123.83, 122.7.

The deuteration rates were calculated based on ¹H NMR data, the deuteration rates of the carbon-hydrogen bonds at different positions in deuterated benzothiophene were obtained as shown in the reaction equation.

### Example 50

### Preparation of Deuterated β-Methylstyrene

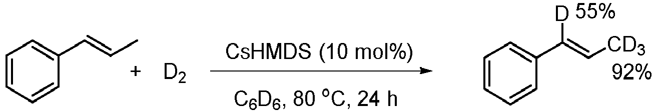

Under an inert atmosphere, in a 25 mL autoclave, β-methylstyrene (CAS: 637-50-3) (0.0355 g, 0.3 mmol), deuterated benzene (0.5 mL) and cesium bis(trimethylsilyl)amide (0.0087 g, 0.03 mmol, 10 mol%) were added sequentially. The autoclave was sealed, filled with 4 bar deuterium gas, heated at 80°C for 24 hours to undergo the deuteration reaction. Then, the reaction solution was directly subjected to ¹H NMR and ¹³C NMR tests. The ¹H NMR spectrum is shown in Figure 52.

The deuteration rates were calculated based on ¹H NMR data, the deuteration rates of the carbon-hydrogen bonds at different positions in deuterated β-methylstyrene were obtained as shown in the reaction equation.

### Example 51

### Preparation of Deuterated 2,3-Dihydrofuran

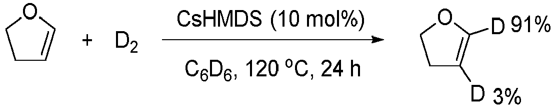

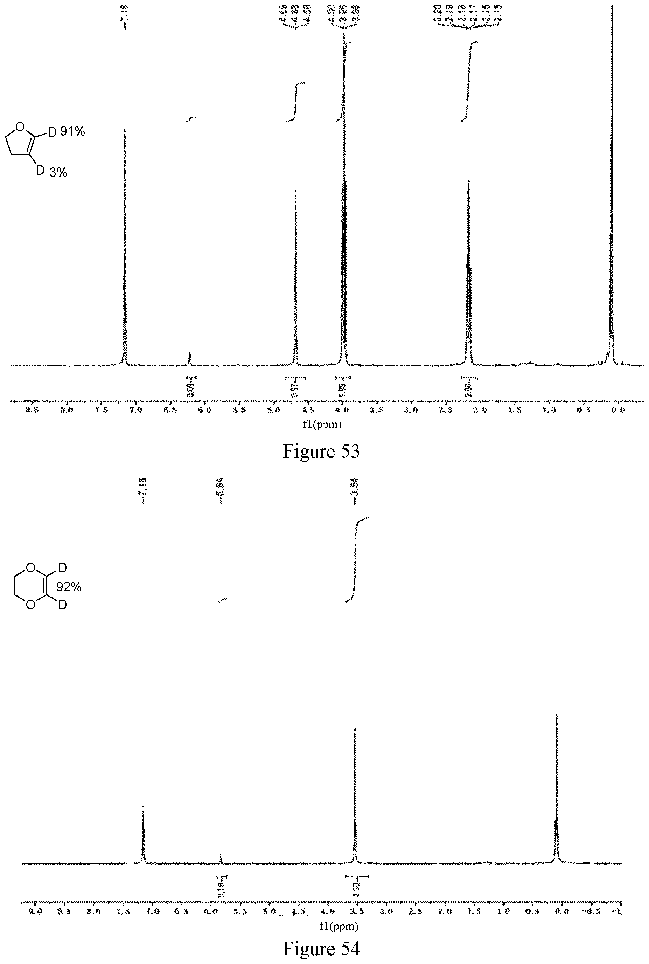

Under an inert atmosphere, in a 25 mL autoclave, 2,3-dihydrofuran (CAS: 1191-99-7) (0.0210 g, 0.3 mmol), deuterated benzene (0.5 mL) and cesium bis(trimethylsilyl)amide (0.0087 g, 0.03 mmol, 10 mol%) were added sequentially. The autoclave was sealed, filled with 4 bar deuterium gas, heated at 120°C for 24 hours to undergo the deuteration reaction. Then, the reaction solution was directly subjected to ¹H NMR and ¹³C NMR testing. The ¹H NMR spectrum is shown in Figure 53.

The deuteration rates were calculated based on ¹H NMR data, the deuteration rates of the carbon-hydrogen bonds at different positions in deuterated 2,3-dihydrofuran were obtained as shown in the reaction equation.

### Example 52

### Preparation of Deuterated 1,4-Dioxene

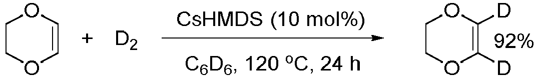

Under an inert atmosphere, in a 25 mL autoclave, 1,4-dioxene (CAS: 543-75-9) (0.0258 g, 0.3 mmol), deuterated benzene (0.5 mL) and cesium bis(trimethylsilyl)amide (0.0087 g, 0.03 mmol, 10 mol%) were added sequentially. The autoclave was sealed, filled with 4 bar deuterium gas, heated at 120°C for 24 hours to undergo the deuteration reaction. Then, the reaction solution was directly subjected to ¹H NMR and ¹³C NMR testing. The ¹H NMR spectrum is shown in Figure 54.

The deuteration rates were calculated based on ¹H NMR data, the deuteration rates of the carbon-hydrogen bonds at different positions in deuterated 1,4-dioxene were obtained as shown in the reaction equation.

### Example 53

### Preparation of Deuterated 1,3-bis-(2,4,6-trimethylphenyl)imidazol-2-ylidene

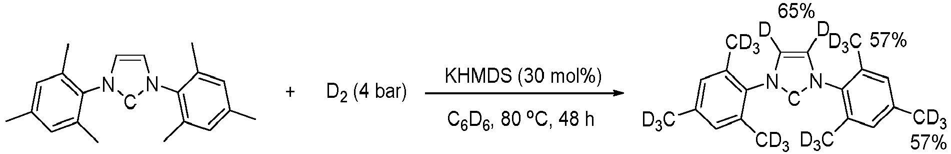

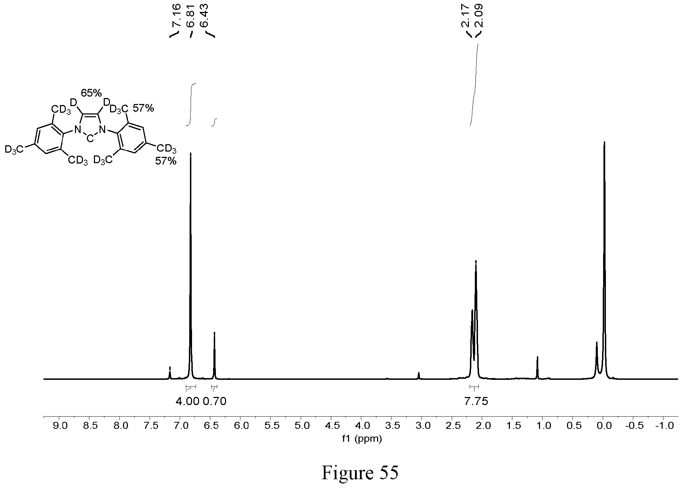

Under an inert atmosphere, in a 25 mL autoclave, 1,3-bis-(2,4,6-trimethylphenyl)imidazol-2-ylidene (CAS: 141556-42-5) (0.0601 g, 0.2 mmol), deuterated benzene (0.5 mL) and potassium bis(trimethylsilyl)amide (0.0120 g, 0.06 mmol, 30 mol%) were added sequentially. The autoclave was sealed, filled with 4 bar deuterium gas, heated at 80°C for 48 hours to undergo the deuteration reaction. Then, the reaction solution was directly subjected to ¹H NMR and ¹³C NMR testing. The ¹H NMR spectrum is shown in Figure 55.

The deuteration rates were calculated based on ¹H NMR data, the deuteration rates of the carbon-hydrogen bonds at different positions in deuterated 1,3-bis-(2,4,6-trimethylphenyl)imidazol-2-ylidene were obtained as shown in the reaction equation.

The above description represents preferred embodiments of the present application and are not intended to limit the scope of the present application. Any modifications, equivalent replacements, improvements, and the like made within the spirit and principles of the present application should be included within the scope of protection of the present application.

## Claims

1. A method for deuteration of a carbon-hydrogen bond, comprising the following steps:
in the presence of a catalyst, introducing deuterium gas into a compound containing carbon-hydrogen bonds for deuteration to produce deuterated compounds;
wherein, said catalyst is selected from at least one of alkali metal hydroxides, alkali metal alkoxides, alkali metal hydrides, alkali metal hydrocarbon compounds, and alkali metal amino compounds.

2. The method of claim 1, wherein,
said alkali metal hydroxide is selected from at least one of lithium hydroxide, sodium hydroxide, potassium hydroxide, rubidium hydroxide, and cesium hydroxide;
said alkali metal alkoxide is selected from at least one of lithium methoxide, lithium ethoxide, lithium isopropoxide, lithium tert-butoxide, sodium methoxide, sodium ethoxide, sodium isopropoxide, sodium tert-butoxide, potassium methoxide, potassium ethoxide, potassium isopropoxide, potassium tert-butoxide, alkoxyl rubidium, and alkoxyl cesium; said alkoxyl rubidium is selected from at least one of rubidium methoxide, rubidium ethoxide, rubidium isopropoxide, and rubidium tert-butoxide; said alkoxyl cesium is selected from at least one of cesium methoxide, cesium ethoxide, cesium isopropoxide, and cesium tert-butoxide;
said alkali metal hydride is selected from at least one of lithium hydride, sodium hydride, potassium hydride, rubidium hydride, and cesium hydride;
said alkali metal hydrocarbon compound is selected from at least one of methyl lithium, trimethylsilylmethyl lithium, ethyl lithium, n-butyl lithium, sec-butyl lithium, tert-butyl lithium, benzyl lithium, phenyl lithium, benzyl sodium, benzyl potassium, benzyl rubidium, and benzyl cesium;
said alkali metal amino compound is selected from at least one of lithium amide, lithium dimethylamide, lithium diethylamide, lithium diisopropylamide, lithium bis(trimethylsilyl)amide, lithium tetramethylpiperidide, sodium amide, sodium diisopropylamide, sodium bis(trimethylsilyl)amide, potassium amide, potassium dimethylamide, potassium diethylamide, potassium diisopropylamide, potassium bis(trimethylsilyl)amide, potassium tetramethylpiperidide, rubidium dimethylamide, rubidium diethylamide, rubidium diisopropylamide, rubidium bis(trimethylsilyl)amide, rubidium tetramethylpiperidide, cesium dimethylamide, cesium diethylamide, cesium diisopropylamide, cesium bis(trimethylsilyl)amide, and cesium tetramethylpiperidide .

3. The method of claim 2, wherein,
said alkali metal amino compound is synthesized in situ using an alkali metal hydrocarbon compound and an amine, or using lithium amide and an alkali metal salt; said alkali metal amino compound is selected from at least one of sodium diisopropylamide, potassium dimethylamide, potassium diethylamide, potassium diisopropylamide, potassium bis(trimethylsilyl)amide, potassium tetramethylpiperidide, rubidium dimethylamide, rubidium diethylamide, rubidium diisopropylamide, rubidium bis(trimethylsilyl)amide, rubidium tetramethylpiperidide, cesium dimethylamide, cesium diethylamide, cesium diisopropylamide, cesium bis(trimethylsilyl)amide, and cesium tetramethylpiperidide;
wherein, said alkali metal hydrocarbon compound is selected from at least one of butyl lithium, benzyl potassium, benzyl rubidium, and benzyl cesium;
said amine is selected from at least one of dimethylamine, diethylamine, diisopropylamine, bis(trimethylsilyl)amine, and tetramethylpiperidide;
said lithium amide is selected from at least one of lithium dimethylamide, lithium diethylamide, lithium diisopropylamide, lithium bis(trimethylsilyl)amide, and lithium tetramethylpiperidide;
said alkali metal salt is selected from at least one of sodium salts, potassium salts, rubidium salts, and cesium salts; said sodium salt is selected from sodium hydride, sodium tert-butoxide; said potassium salt is selected from potassium hydride, potassium tert-butoxide; said rubidium salt is selected from at least one of rubidium fluoride, rubidium chloride, rubidium bromide, rubidium iodide, rubidium nitrate, rubidium sulfate, rubidium carbonate, rubidium formate, rubidium acetate, and rubidium perchlorate; said cesium salt is selected from at least one of cesium fluoride, cesium chloride, cesium bromide, cesium iodide, cesium nitrate, cesium sulfate, cesium carbonate, cesium formate, cesium acetate, and cesium perchlorate.

4. The method of claim 3, wherein, said compound containing a carbon-hydrogen bond is selected from at least one of the following: aromatic compounds with carbon-hydrogen bonds at the benzyl position; thioether compounds with carbon-hydrogen bonds at the α position; sulfone, sulfoxide, sulfonamide, and sulfinamide compounds with carbon-hydrogen bonds at the α position; aryl ether compounds, fluorobenzene compounds, aromatic hydrocarbons, heteroaromatic hydrocarbons with aromatic carbon-hydrogen bonds; and alkene compounds with alkenyl carbon-hydrogen bonds.

5. The method of any one of claims 1-4, wherein, said deuteration reaction is shown in any one of Reaction Equation 1 to Reaction Equation 5;
Reaction Equation 1 is: 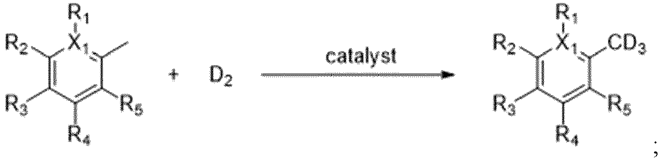
Reaction Equation 2 is: 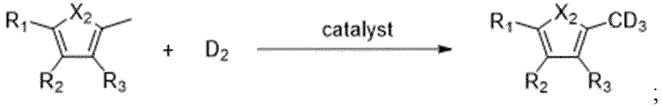
R₁ to R₅ are independently selected from any one of H, C₁-C₁₀ hydrocarbon group, C₆-C₂₀ aryl group, halogen, C₁-C₁₀ hydrocarboxyl group, C₃-C₉ silyl group, and C₁-C₂₀ amino group, two adjacent groups in R₁-R₃ can be connected into a ring, X₁ is a carbon atom or a nitrogen atom, and X₂ is an oxygen atom, a sulfur atom, or a nitrogen atom;
Reaction Equation 3 is: 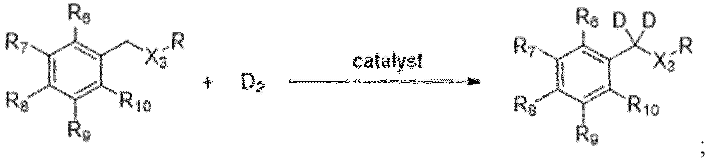
Reaction Equation 4 is: 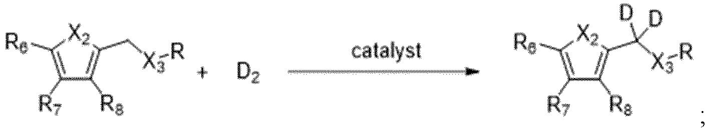
R₆ to R₁₀ are independently selected from any one of H, C₁-C₁₀ hydrocarbon group, C₆-C₂₀ aryl group, halogen, C₁-C₁₀ hydrocarboxyl group, or C₁-C₂₀ amino group, two adjacent groups in R₆-R₈ can be connected into a ring, X₂ is an oxygen atom, a sulfur atom, or a nitrogen atom, and X₃ is selected from any one of a silicon atom, an oxygen atom, a sulfur atom, a carbon atom, or a nitrogen atom; R is a C₁-C₁₀ hydrocarbon group or a C₆-C₂₀ aryl group;
Reaction Equation 5 is: 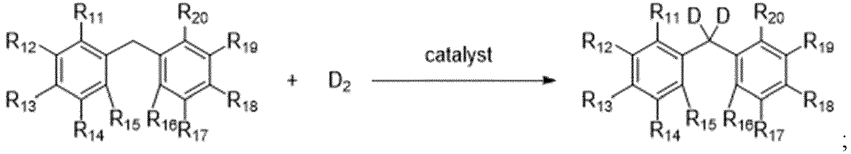
R₁₁ to R₂₀ are independently selected from any one of H, C₁-C₁₀ hydrocarbon group, C₆-C₂₀ aryl group, halogen, C₁-C₁₀ hydrocarboxyl group, or C₁-C₂₀ amino group.

6. The method of any one of claims 1-4, wherein, said deuteration reaction is as shown in any one of Reaction Equation 6 to Reaction Equation 7:
Reaction Equation 6 is: 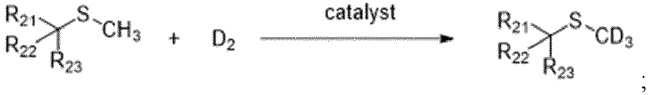
R₂₁ to R₂₃ are independently selected from any one of H, C₁-C₁₀ hydrocarbon group, C₆-C₂₀ aryl group, C₃-C₉ silyl group, C₁-C₁₀ alkoxyl group, or C₁-C₂₀ amino group, R₂₁ to R₂₃ can be connected into aliphatic hydrocarbon rings or aromatic rings;
Reaction Equation 7 is: 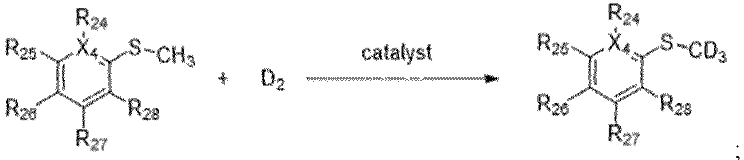
R₂₄ to R₂₈ are independently selected from any one of H, C₁-C₁₀ hydrocarbon group, C₆-C₂₀ aryl group, halogen, C₁-C₁₀ hydrocarboxyl group, C₁-C₁₀ hydrocarbon sulfur group, or C₁-C₂₀ amino group, X₄ is a carbon or nitrogen atom.

7. The method of claim 5 or 6, wherein, said catalyst is selected from at least one of potassium diisopropylamide, potassium bis(trimethylsilyl)amide, potassium tetramethylpiperidide, rubidium diisopropylamide, rubidium bis(trimethylsilyl)amide, rubidium tetramethylpiperidide, cesium diisopropylamide, cesium bis(trimethylsilyl)amide, and cesium tetramethylpiperidide.

8. The method of any one of claims 1-4, wherein, said deuteration reaction is as shown in any one of Reaction Equation 8 to Reaction Equation 12;
Reaction Equation 8 is: 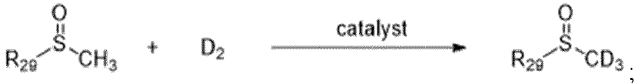
Reaction Equation 9 is: 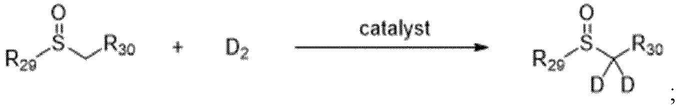
Reaction Equation 10 is: 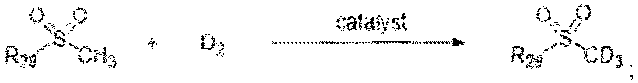
Reaction Equation 11 is: 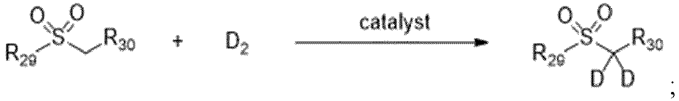
Reaction Equation 12 is: 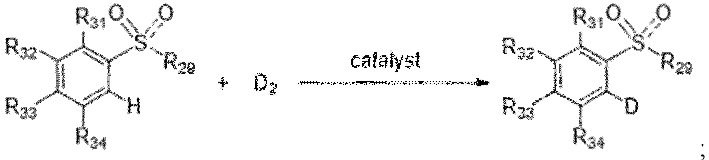
R₂₉ is independently selected from any one of C₁-C₁₀ hydrocarbon group, C₆-C₂₀ aryl group, C₁-C₁₀ alkyloxyl group, or C₁-C₂₀ amino group, R₃₀ is independently selected from any one of C₁-C₁₀ hydrocarbon group or C₆-C₂₀ aryl group, and R₂₉, R₃₀ can be connected into a ring; R₃₁ to R₃₄ are independently selected from any one of H, C₁-C₁₀ hydrocarbon group, C₆-C₂₀ aryl group, C₁-C₁₀ alkyloxyl group, or C₁-C₂₀ amino group, and two adjacent groups among R₃₁ to R₃₄ can be connected into a ring.

9. The method of claim 8, wherein, said catalyst is selected from at least one of potassium tert-butoxide, sodium hydroxide, or potassium hydroxide.

10. The method of any one of claims 1-4, wherein, said deuteration reaction is as shown in any one of Reaction Equations 13 to 15;
Reaction Equation 13 is: 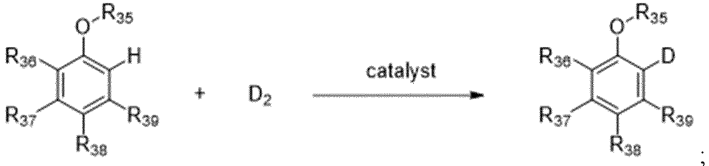
Reaction Equation 14 is: 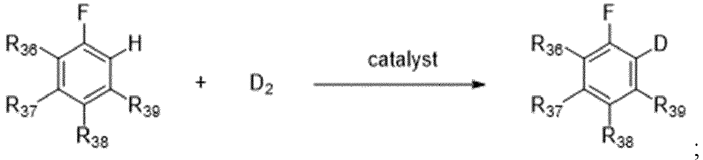
Reaction Equation 15 is: 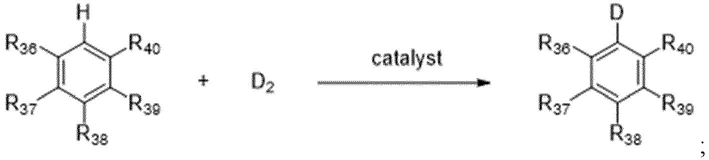
R₃₅ to R₄₀ are independently selected from any one of H, C₁-C₁₀ hydrocarbon group, or C₆-C₂₀ aryl group, and two adjacent groups among R₃₅ to R₄₀ can be connected into a ring.

11. The method of any one of claims 1-4, wherein, said deuteration reaction is as shown in any one of Reaction Equation 16 to Reaction Equation 17;
Reaction Equation 16 is: 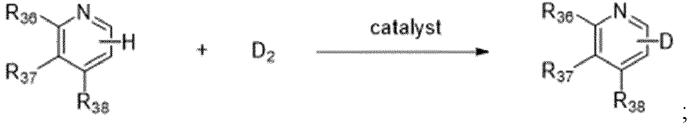
Reaction Equation 17 is: 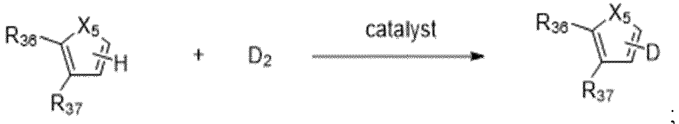
R₃₆ to R₃₈ are independently selected from any one of H, C₁-C₁₀ hydrocarbon group, or C₆-C₂₀ aryl group, and two adjacent groups among R₃₆ to R₃₈ can be connected into a ring, X₅ is selected from any one of a silicon atom, an oxygen atom, a sulfur atom, a carbon atom, or a nitrogen atom.

12. According to any one of claims 1-4, wherein, said deuteration reaction is as shown in Reaction Equation 18:
Reaction Equation 18 is: 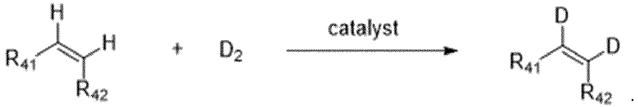 ,
R₄₁ and R₄₂ are independently selected from any one of H, C₁-C₁₀ hydrocarbon group, C₆-C₂₀ aryl group, C₃-C₉ silyl group, C₁-C₁₀ alkoxyl group, or C₁-C₂₀ amino group, and R₄₁ and R₄₂ can be connected into an aliphatic hydrocarbon ring or an aromatic ring.

13. The method of any one of claims 10-12, wherein, said catalyst is selected from at least one of potassium diisopropylamide, potassium bis(trimethylsilyl)amide, potassium tetramethylpiperidide, rubidium diisopropylamide, rubidium bis(trimethylsilyl)amide, rubidium tetramethylpiperidide, cesium diisopropylamide, cesium bis(trimethylsilyl)amide, and cesium tetramethylpiperidide.

14. The method of any one of claims 1-13, wherein, the molar amount of said catalyst is 1%-100% of the molar amount of said compound containing carbon-hydrogen bonds.

15. The method of any one of claims 1-14, wherein, the pressure of deuterium gas in said deuteration reaction is between 1 bar-50 bar.

16. The method of any one of claims 1-15, wherein, said deuteration reaction is conducted in an organic solvent or under solvent-free conditions, and said organic solvent is selected from at least one of deuterated benzene, benzene, tert-butylbenzene, n-hexane, cyclohexane, decalin, ether, tetrahydrofuran, methyl tert-butyl ether, methyl cyclopentyl ether, and n-butyl ether.
